# EUROPEAN PATENT SPECIFICATION

(11) **EP 2 249 695 B1**
(45) Date of publication and mention of the grant of the patent: **20.04.2016**
(21) Application number: 09715287.0
(22) Date of filing: 26.02.2009
(51) Int. Cl.: A61B 5/151, A61B 5/145, A61B 5/157

(54) **SYSTEM FOR MODULAR ANALYTE MONITORING**
SYSTEM FÜR MODULARE ANALYTÜBERWACHUNG
SYSTÈME DE SURVEILLANCE MODULAIRE DE SUBSTANCE À ANALYSER

(30) Priority: 27.02.2008 US 67424 P; 27.02.2008 US 67423 P; 19.05.2008 US 54180 P; 05.06.2008 US 58938 P; 08.06.2008 US 59773 P; 07.09.2008 US 94915 P; 07.09.2008 US 94916 P
(43) Date of publication of application: 17.11.2010
(73) Proprietor: Mon4d Ltd., Tel-Aviv 62502 (IL)
(72) Inventor: PESACH, Benny, 48072 Rosh-Ha'ayin (IL); BITTON, Gabriel, 97279 Jerusalem (IL); NAGAR, Ron, 62502 Tel-Aviv (IL)
(74) Representative: Giles, Ashley Simon
(86) International application number: PCT/IL2009/000221
(87) International publication number: WO 2009/107135

(56) References cited:
- WO-A2-2007/064596
- US-A1- 2004 010 207
- US-A1- 2006 094 985
- US-A1- 2006 235 285
- US-A1- 2007 166 196
- US-A1- 2007 270 672
- US-B1- 6 558 321

## Description

### FIELD OF THE INVENTION

The present invention relates to a system for measuring an analyte from blood.

### BACKGROUND OF THE INVENTION

Diabetes is a very serious illness affecting millions of people. Many diabetic users are required to measure their glucose level 5-7 times a day to maintain proper blood glucose levels. Currently, individuals intermittently measure capillary blood glucose levels by extracting a few drops of blood using what are known as finger sticks. However, intermittent use is still based on a manual operation and handling of a lancet to prick the skin and the introduction of a "glucose test strip" to the drop of blood which comes out and performs a measurement of the blood glucose level. The procedure is manual, cumbersome and may be difficult to perform by young kids and the elderly. The procedure takes time and has to be performed carefully so as to ensure the accuracy of the measurement and to prevent the introducing of measurement errors due to operator. Additionally these devices require the operator to be awake in order to manipulate, work and/or activate the device.

Automatic glucose measurement can be performed by a new generation of continuous glucose monitors which allow individuals to continuously measure glucose levels. Some continuous glucose monitors include one or more sensors that are inserted or implanted into a blood vessel such as the vena cava. However, implanting a sensor into a blood vessel is a complicated process that currently requires a surgical procedure and involves potential severe adverse effects.

State of the art substance sensors are typically inserted into subcutaneous tissue. For example, the Guardian RT by Medtronic, the Navigator by Abbott and the STS by Dexcom measure glucose levels in interstitial tissue with such subcutaneous sensors. Such sensors comprise a selectively permeable membrane that allows glucose to flow through to an enzymatic assay that determines the relative glucose level.

Alternatively, the subcutaneous sensors include the microdialysis catheter substance sensors, such as for example Menarini GlucoDay for glucose measurement or the device of CMA microdialysis Microdialysis device Corp (USA) for measurement of several substances. In microdialysis the substance molecules diffuse into the catheter membrane and flow, optionally by use of a pump, to an external sensor, instead of inserting a sensor into the tissue, as is done in the subcutaneous biosensors.

Continuous Glucose Monitoring (CGM) sensors operate automatically to provide glucose readings after an initial calibration process and can also provide measurements during the time that the user is asleep. However, none of the Continuous Glucose Monitoring systems (CGM) are approved for primary or independent use by the regulatory authorities, such as the FDA and they are only approved for adjunctive use. The main reason for that is their relatively low accuracy, especially at low glucose levels when compared to glucose reading in samples taken from blood. Another limitation of the CGM is a change in their performance over time. Upon installation, the CGM is calibrated against measurement from blood samples from the finger using regular glucose meters. Based on these measurements and the sensor reading a calibration factor is calculated.

State of the art blood analyte measuring systems can be for example found in US2006/0094985A1 and US2004/0010207A1.

### SUMMARY OF THE INVENTION

There is an unmet need for and it would be highly useful to have, an automatically and/or remotely operated modular analyte measuring device, system and a method for monitoring an analyte from a bodily fluid.

The present invention overcomes the drawbacks of the background art by providing an analyte measuring system according to claim 1.

Preferably the device provides for measuring at least one or more analytes in an automatic predetermined manner while optionally and preferably determining an action to be performed based on such monitoring. Optionally the device may also be linked to a plurality of devices to form a closed loop system.

The term "bodily fluids" within the context of this application refers to any biological bodily fluid, preferably blood, but for example also including but not limited to interstitial fluid (ISF), saliva, urine or the like to measure an analyte diffused therein. A preferred and most favorable bodily fluid is blood.

The term "analyte" within the context of this application preferably refers to glucose, but also refers to a measurable biological entity, peptide, fat, molecule, chemical, compound, solution, gas, carbohydrate or the like measured that may be detected from bodily fluid. Optionally and preferably the term analyte refers to glucose used for monitoring the insulin levels within the body. Optionally, the analyte may be cholesterol and or triglycerides for monitoring atherosclerosis.

The term "closed loop analyte monitoring" refers to a system and its components working together synergistically to provided control over analyte levels in a flowing bodily fluid, for example blood. For example, diabetics need to closely control their blood glucose levels. In order to achieve this control, a closed loop system is needed to maintain baseline blood glucose levels. Effectively, a closed loop monitoring system continuously measures blood glucose levels while providing an measured response in relation to any changes in the blood glucose levels, preferably by delivering the appropriate amount of insulin (or other substance in order to reduce blood glucose level) and possibly glucagon (or other substance in order to increase blood glucose level). Therefore a closed loop system within the context of diabetes comprises cycles of measuring and or inferring blood glucose levels and delivering relative amounts of insulin. Similar closed loop system for other measurable analytes for example cholesterol, hormone levels may be implemented with the device, system and method.

The term piercing element within the context of this application refers a mechanical device comprising a piercing and or cutting surface for example including but not limited to a lancet, blade, blade edge, sharp edge, needle, sharp point, scoring edge, tapered edge, cross shaped blade or the like for piercing the skin. Preferably, the skin is pierced to draw or release bodily fluids for analysis and/or measurement. Optionally a piercing element may comprise non mechanical instrumentation for piercing tissue for drawing a biological fluid for example including but not limited to laser, acoustic, piezoelectric, electrical, optical or the like technologies as is known and accepted in the art.

A preferred embodiment provides for a method and device for automatic high reliability measurement of an analyte from a bodily fluid for example including but not limited to blood, urine, saliva, interstitial fluid or the like to measure glucose and/or other analyte levels.

Within the context of this application the term "analyte measuring medium" is interchangeably used with the terms, "analyte measuring strip", "test strip", finger sticks or the like to refer to the analyte measuring medium as are known and accepted in the art. In some embodiments specialized improvements of such analyte measuring mediums are disclosed, for example providing improved bodily fluid collection, and improved coagulation.

A preferable embodiment provides for a wearable analyte measuring device that is adept to measuring an analyte, most preferably glucose, from a bodily fluid, most preferably blood, in seamless manner even during sleep. Most preferably the analyte measuring device may be coupled to the skin surface on an extremity for example along the arms and or legs. Optionally the analyte measuring device may be worn on an extremity for example including a finger tip, finger along its length, toe or the like.

An optional embodiment for an analyte measuring device enables at least one and more preferably a plurality of measurements to be performed according to a predetermined number of measurements or controllable time frame, period, schedule and/or time interval. Optionally and preferably, such control is remotely provided by an external or remote triggering device.

Most preferably the analyte measuring device may be attached to the skin of a user while the result of the measurements may be communicated to at least one of the device itself, a local device, a remote device, or any combination thereof.

An optional embodiment of the present invention provides for an analyte measuring device and system wherein the measuring device may be remotely controlled. Optionally and preferably, remote control for example includes but is not limited to communicating a triggering command to initiate the device according to an optional embodiment of the present to undertake analyte measuring. Optionally, the results of such analyte measuring may be further communicated to at least one of the triggering source, a calling center, local display, a back office, a remote location or the like or any combination thereof.

A preferred embodiment for an analyte measuring device preferably utilizes and comprise modified and device specific analyte measuring medium also referred to an analyte measuring elements for example including but not limited to test strips and/or finger sticks as known and accepted in the art.

A preferred embodiment provides for an analyte monitoring device comprising at least two or more portions, wherein a first portion also referred as the reusable portion, is configured to be reusable allowing for repeated use while a second disposable portion is configured to be disposable most preferably for single time use. Most preferably disposable portion and reusable portion may be readily and securely associated or disassociated with one another. Optionally and preferably the secure association or disassociated is facilitated by the use of connectors that may be functionally manipulated to associate or disassociate a reusable portion and a disposable portion.

Most preferably, the disposable portion comprises disposable elements and parts that are most preferably adept for single or optionally limited use that are preferably associated with obtaining and/or sampling a bodily fluid, for example comprising a piercing element and test strip.

Most preferably the reusable portion is configured to provide at least one or more state of the art sensors or the like for measuring the sampled bodily fluid, for example including but not limited to amperemetric, acoustic, ultrasonic, optical, electromagnetic, infrared or the like systems or methods for measuring an analyte from a bodily fluid as is known and accepted in the art.

Optionally the reusable portion is configured to act as a power source to at least one or more of its optional components for example including but not limited to a controller, a motor for driving the disposable portion, an analog to digital converter, and a transponder to transmit data and receive commands from a remote unit and/or controller.

Optionally disposable portion and/or reusable portion may be adapted to accepted interchangeable faces for example including decorative faces.

Most preferably, the device may be activated remotely via communication protocols known and accepted in the art for example including but not limited to contactless, RF, wireless, cellular, IR, Bluetooth or the like communication protocols.

Most preferably, the device may further comprise a communication module providing for two way communication, for example both receiving data and sending data using communication protocols known and accepted in the art for example including but not limited to contact-less, RF, wireless, cellular, Bluetooth, IR or the like communication protocols.

Optionally, the analyte measuring device may comprise at least one or more tissue treatment elements for improving analyte measurement and/or analysis. Optionally, at least one or more tissue treatment element may optionally be used for example including but not limited to heating, mechanical massage, suction, electrical energy, ultrasound energy, optical energy, acoustic energy, vasodilatation drugs or the like.

Most preferably the analyte measuring device is realized as a modular analyte monitoring device that may be a member of a greater system for closed loop monitoring of an analyte sampled from a bodily fluid, most preferably blood. Optionally and preferably the continuous monitoring system comprises active control of the three primary monitoring facets including continuous monitoring, continuous response - optionally drug response- and continuous centralized processing and control. Optionally, the device can directly interface, sync and link with a central controller device. Optionally, the device can interface and interact with a plurality of devices within the closed loop system. Optionally, a plurality of modular devices may interface with one another optionally using a communication port.

In some embodiments there are more pairs of piercing element and analyte measurement element disposed within a single disposable unit, such that a plurality of measurements may be undertaken with the same analyte measuring device, for example located on the user's finger. For example, a multi use disposable analyte measuring device providing automatic predetermined or seamless measurement that may be particularly useful for infants, ambulatory applications, the disabled and or handicapped, or otherwise dependent user.

An optional embodiment provides for a specialized analyte measuring element or medium comprising an analyte measurement channel along with at least one or more secondary functional areas, for example a blood coagulation element preferably to prevent excess bleeding at the piercing site. Optionally the functional elements may be disposed on the same surface or on opposite surfaces of the measuring element. Optionally blood coagulation element may take various forms for example including but not limited to a gauze pad, band aid, medicated pad with a coagulating agent, or the like elements for preventing excess blood loss and absorbing excess pooled blood.

In some embodiments the measuring unit may contain an optical sensor which can be used to measure if enough blood accumulated before advancing the strip to the blood pooling area. If a large enough blood sample exists on the skin the strip advances and performs the measurement. If the blood sample is not large enough the strip will not advance and other or additional methods will be employed to extract more and/or sufficient bodily fluid, most preferably blood before advancing the strip.

Optionally, a sensor may be utilized to determine if sufficient bodily fluid has been extracted. Optionally the sensor may be disposed either in the disposable portion or most preferably in the reusable portion. Optionally, the sensor may be composed of a light source at a wavelength which is absorbed strongly by hemoglobin and a light detector. The light source and light detector are aligned so that the light from the light source will be reflected from the skin piercing area into the light sensor. The skin is a good scattering surface, however, when that area is covered with enough blood the reflected signal will be strongly attenuated due to the hemoglobin absorption of light. When the light reading is below a certain level it indicates that a large enough blood sample is present on the skin.

Optionally, plurality of sensors with or without optical element can be used to measure the size and or volume of the extracted bodily fluid, such as blood. For example a tiny CMOS camera can be used to measure the size of the extracted blood drop.

In some embodiments the analyte measuring strip might be advanced parallel to the skin and in contact with the skin to allow for the strip to collect blood scattered along the path of the advancing strip. The strip opening where the bodily fluid, most preferably blood, is absorbed into the strip is typically a 4 mm wide. The strip path can be long, for example as much as 5mm. Hence the strip can collect blood sample from an area of about 20 mm^2 (squared) which improves the blood collection efficiency and enhances the likelihood of collecting a sufficiently large sample for analyte measurement.

In an optional embodiment the piercing element and or analyte measuring device may be housed in a small hollow cylinder. Preferably the cylindrical housing may be composed of at single disposable unit. Optionally, the cylindrical housing may be composed of two or more portion having a first disposable portion and a second reusable portion. Optionally, the cylindrical housing may be composed of two disposable portions. Optionally, the cylindrical body may be disposed of expandable and/or rotatable elements providing for a housing comprising varying diameter. Optionally, the cylindrical body is elastic and or flexible.

In one preferred embodiment the user may choose to have at least one and more preferably a plurality of measurements taken at one at a time (wherein each measurement is at a different time), and a different number of measurements taken at another time. Preferably the user can depict the number of measurements to be performed by adding or removing the number of disposable and/or reusable elements operated by the same remote controller. Preferably the controller can operate a plurality of measuring elements optionally based on predetermined and/or schedule, or based on a preset time interval, or based on the receipt of a triggering command to undertake measurement, or optionally by coordinating and monitoring the operational activity and/or status of a plurality of measuring elements simultaneously.

A further preferred embodiment relates to systems and methods for measuring and monitoring an analyte in a bodily fluid. Optionally and preferably the system comprises at least one and more preferably a plurality of modular analyte measuring device as previously described and at least one or more controller. Optionally, the system comprises up to about 10 modular analyte measuring devices simultaneously in use with the controller.

Most preferably, the modular analyte measuring device comprises two portions a reusable portion and a disposable portion, as previously described. Most preferably, the disposable portion is configured for single use comprising a single use piercing element and analyte measuring medium. Preferably, reusable portion comprising a battery may be synced with the controller for recharging batteries. Most preferably, the reusable portion of the measuring device comprise communication module to provide for receiving and sending data relating to the analyte measurement. Communication protocols received from the controller for example includes but is not limited to triggering data, alarms, scheduling or the like instructions. Optionally and preferably, the reusable portion sends the measurement results to the controller, alarms, status data or the like information. Most preferably communication for either receiving or sending comprises at least one or more or a combination of communication protocols as is known and accepted in the art for example including but not limited to wired, physical, contactless, wireless, cellular, IR, RF, optical, Bluetooth or the like communication protocol.

Optionally and preferably, the controller may provide and/or display information regarding the analyte measuring device for example including, ready state, battery state, schedule timing, measurement status, measurement results, alarms or the like data. Most preferably ready state of the analyte measuring device is communicated once the reusable and disposable portions are securely associated and functional. Optionally the controller provides a unique identification label for each analyte measuring device it is associated and/or in communication with. Optionally and preferably the analyte measuring device may communicate to at least one or more controllers its state in relation to the user, for example indicating if the measuring device has been attached to a user and is in a ready to use format or similarly if the device is in the midst of obtaining a measurement.

Most preferably, the controller may be realized in at least one and optionally a plurality of associated units. Optionally the controller may be realized in the form of a remote unit and/or a proximal unit. Optionally and preferably the remote unit comprises communication protocols adept for long range wireless communication for example including but not limited to cellular, wireless, RF, IR, optical or the like long range communication protocols. Most preferably, the remote may be located at some distance from the analyte measuring device of the present invention while still maintaining communication channels enabling sending and/or receiving long range instructions.

Optionally and preferably the proximal unit comprises communication module adept for short range communication protocols for example including but not limited to Bluetooth, contactless RFID, IR, wired or the like communication protocols. Most preferably, the proximal controller unit may be located in the vicinity of the analyte measuring device according to a preferred embodiment while still maintaining communication channels with at least one or both of the analyte measuring device and the remove controller unit.

Optionally, the remote and/or proximal units may be configured to securely associate or disassociate with one another preferably forming a single housing. Optionally, proximal and remote device may comprise controllable configurations for example master and slave, wherein most preferably the remote unit is the master. Optionally the controller, in either the proximal or remote configuration may be realized in the form of a PDA, cellular phone, mobile computer or the like processing tool. Optionally and preferably, the controller comprises, a display, for example an LCD screen, LED array, or the like. Optionally and preferably, the controller comprises user interface for setting and controlling its activity. Preferably, the controller comprises a speaker, user interface ports, charging port contactless port, sync port to associate with the reusable portion.

Most preferably alarm state is communicated to all modules comprising the system.

In one preferred embodiment wherein the disposable element comprises a single lancet and a single test strip are housed together in a housing having one side that can be securely connected to the skin of a user, optionally human or animal. The housing is preferably further mechanically and electrically connected to a reusable element comprising a power source, controller, transponder and mechanical actuators. Optionally the combined measuring element comprising a reusable and disposable portion securely associate with one another to form an active measuring device also referred to as a measuring "button" having a size of about 10mmx10mmx5mm. Most preferably, at least one or more of the measurement "buttons" may be attached to the user at the same time one time according to a predetermined number of measurements to be taken. Optionally and preferably a remote controller which can optionally take the form of a cellular phone may be used to control the measuring button's controller to trigger a single measurement at a time. Optionally and preferably the triggering command to perform a measurement may originate in plurality of sources for example including but not limited to be preset schedules, a preset specific time, a time intervals based on pervious measurements. Optionally a triggering command may optionally be received via mobile telephone communication for example including an SMS that is converted to a triggering command. Optionally the triggering command and/or mobile communication trigger may be received from an alternate cell phone and/or command center. Most preferably, each such measuring button performs only one measurement.

In yet another preferred embodiment the modular system comprising several "measuring buttons" such that one or more measuring buttons is placed on one body part (arm, leg) while one or more other measuring buttons are laced on a different body part (opposite arm, opposite leg).

In yet another preferred embodiment the system controller receive signal from a device that is placed on the user (on the skin or implanted) and in return initiates a measuring button to perform a measurement. The device that is placed on the skin or implantable can detect a physiological parameter or tissue analyte (like ISF glucose level, or eating activity).

An optional embodiment of the system may be configured to comprise at least one and more preferably a plurality of modular analyte measuring device as previously described, at least one controller as previously described and a treatment and/or drug delivery device.

Optionally and preferably a treatment and/or drug delivery device for example including but not limited to a drug delivery pump, a device for providing a treatment or medicament in light of a measured and /or sensed analyte levels. Most preferably, the drug delivery device is controlled by the controller wherein the dosage form, dosage amount or the like is determined and communicated to the drug delivery device.

Most preferably alarm state is communicated to all modules comprising the system.

An optional embodiment provides for a system utilizing continuous glucose sensor, for example including but not limited to an ISF Glucose Sensor (IGS), and Automatic Blood Glucose Sampling (ABGS) system as described with regard to some embodiments of the present invention or the like as is known in the art. Optionally an IGS and ABGS may be coupled to , a treatment device such as an insulin pump and controller.

Most preferably, the automatic glucose sensor continuously senses the glucose levels, from at least one or more preferably a plurality of sources for example including invasive measurements from a bodily fluid preferably ISF, but for example also including, blood, saliva, urine or the like, or form non-invasive measurements.. Optionally, the ABGS is triggering by a change in the actual glucose levels or in the rate of change of the glucose levels, according to a schedule, or any combination thereof.

Optionally and preferably, the system according to the present embodiment communicates with a controller with at least one or more communication protocols as is known and accepted in the art for example including but not limited to contactless, wired, wireless, RF, IR, Bluetooth, cellular or the like. Optionally, the controller may be embodied in a processor, mobile phone, PDA, computer or the like.

Optionally and preferably, the system may further comprise a treatment indication and/or device for indicating or carrying out a treatment. For example including a pump and or infusion set may be utilized for administering and/or delivery a medicament or the like non-medicinal fluid, for example saline, glucose or the like. Optionally, treatment indication may be utilized to indicate to an appropriate course of action, for example self delivery of a medicament or food.

In some embodiments the ABGS can save at least one or more manual blood sampling currently needed to calibrate the IGS, sensors known in the art. Currently, the IGS technologies in the market require few calibrations with blood glucose readings. For instance, the Gaurdian RT requires calibration point 2 hours after the sensor insertion and later on every 12 hours. An IGS combined with ABGS can improve compliance comparing to the regular IGS and reduce the burden to the IGS user, by taking the blood glucose sampling automatically as required. In some embodiments, to get better accuracy of the IGS the calibration points may optionally and preferably be undertaken at specific conditions, such as at relatively stable glucose levels, such as <0.5mg/dl/min glucose change, for period longer than 20min, to minimize the difference between ISF and blood glucose levels, which become larger during larger variations of the glucose level. In this way, a better calibration accuracy of the IGS can be achieved and reduce the overall error of the IGS reading compared to blood glucose level. An optional good calibration point can be during the evening or at night when the user sleeps, after the postprandial glucose variation associated with the users food intake has passed. According to the present embodiment the ABGS can take the blood glucose measurement automatically, while the user sleeps.

Some embodiments are particularly adept in dealing with important medical decision required due to low blood glucose levels that are in danger of reaching hypoglycemic levels a (blood glucose level <60mg/dl) at a point where the user most preferably requires a quick increasing their blood glucose levels optionally by consuming carbohydrate or glucose rich foods or drinks having high glucose concentrations to elevate the blood glucose level to the normal glucose level (80-110mg/dl). Currently, IGS devices fail to provide good enough alarm for hypoglycemia because of their accuracy limitations, especially at low glucose level. An optional embodiment provides for an additional automatic blood sample at instances of low glucose levels to overcome the IGS inaccuracy problem therein providing an accurate reading of the blood glucose at such critical times. Accordingly an optional embodiment can act as a support to improve the hypoglycemic alarm most preferably to level of no false alarms therein preferably facilitating any medical decision that may optionally be derived at, such as glucose or carbohydrates consumption.

Another optional embodiments provides for improved ISG calibration during a user's physical activity preferably by triggering an analyte measurement during such physical activity. It is known that activity level may alter the reading of the ISG sensor and destroy the calibration. The ABGS can be triggered and used to improve ISG sensor reading therein restoring calibration. In some embodiment the level of activity may be determined based on a gyro-meter and/or accelerometer placed on the ABGS or on the ISG sensor to trigger the activity of the analyte measuring device (ABGS).

In some embodiments the initiation of the ABGS is based on the subject skin temperature. It is known that the skin and body temperature may alter the reading of the ISG sensor and destroy the calibration. The ABGS can be triggered to improve ISG sensor reading and restore calibration. In some embodiments the skin temperature may be determined by a temperature sensor placed on ISG sensor.

In some embodiments the ABGS is initiated when the IGS reading drops below a certain level, such as 70mg/dl. In this case some automatic blood glucose measurements may be initiated even though the real glucose level is higher, which means that the user is not in a hypoglycemic state however this goes undetected with an IGS readings alone. An optional embodiment the IGS sensor may be recalibrated based on the blood glucose levels detected by the ABGS of the present invention to ensure accurate IGS tracking the glucose level. In some embodiments, if the IGS reading further decrease another automatic blood glucose measurement can be initiated and the process can repeat until the glucose level goes up to the normal level. Optionally, the threshold for initiating an automatic blood glucose measurement can be set to determine the trigger level.

In some embodiments the ABGS is initiated when the IGS reading drops below a certain level, such as 80mg/dl accompanied glucose decrease rate above a certain level, for example above 2mg/dl/min. The combination of rapid glucose decrease and relatively low glucose level will trigger the ABGS to avoid un-safe state which may result from the combination of inaccuracies due to, rapid glucose change and relatively low glucose level. An algorithm can be used to determine if a certain state (combination of glucose level and glucose change rate) triggering of the ABGS to improve accuracy. Rapid glucose decrease will require initiation of the ABGS at higher glucose level. For example rapid glucose change above 3 mg/dl/min will induce initiation of the ABGS at glucose level such as 90 mg/dl.

In some embodiments the ABGS is initiated when the IGS reading drops or increased too rapidly, such as in a rate larger than 2mg/dl/min, where the accuracy of IGS readings is known to be too low. In this case the IGS readings can be recalibrated according to the ABGS reading and the IGS can keep tracking the glucose level and/or alert the user as required.

In some embodiments the ABGS is initiated in certain time points after the IGS reading starts to increase at a rate higher than a certain rate, such as 1mg/dl/min, and indicating that the user had a meal or consumed some carbohydrates. Currently, most diabetic users have large postprandial glucose excursions because of the large difference between the desired effect of the insulin and the actual effect which depends on many parameters currently not available to the user, such as the temporal insulin resistance and the gastric absorption time constant of the carbohydrates. Such large postprandial glucose excursions can be reduced by measuring the postprandial glucose level after certain periods from the meal start and fixing the insulin dose accordingly. However, the accuracy of the IGS readings is not accurate and reliable enough for calculation of the required insulin dose adjustment. By adding an ABGS sampling the problem of the IGS accuracy can be solved. For instance the glucose level can be measured 1 hour or 2 hours after the meal starts and the insulin bolus starts its action. Preferably in this case an algorithm that may take into account also additional parameters, such as consumed carbohydrates, the infused insulin history and the user sensitivity to insulin could optionally and preferably provide the adjustment to the insulin dose in order to reduce the postprandial glucose excursions.

In case of using rapid acting insulin, such as the insulin analogues or even faster acting insulin such as the ViaJect or using a drug delivery device that improves the insulin absorption or intradermal delivery , the insulin clearance from the tissue is faster then in the regular insulin. In those cases it may be more feasible to take additional glucose measurement and adjust the insulin dose to regulate the blood glucose level, since the insulin residual concentration in the tissue is very small and it is easier to calculate the additional dose that is required for regulating the glucose level.

In some embodiments the ABGS is initiated when the IGS reading drops below a certain level, such as 70mg/dl at night. In this case the automatic blood glucose measurements may be initiated to get a more accurate and reliable glucose reading that if confirm that the glucose level getting low it may initiate an immediate action to correct that. In some embodiments the device may communicate with the insulin pump and stop or decrease or recommend decreasing the basal insulin delivery. In some embodiments the device may alert the user that the glucose level is getting low and wake him up as described at several configurations at the present application. In some embodiments, such as in young diabetic users, the device may alert a third party, such as the user's parents and wake them up, so they can give the diabetic user the required treatment. In some embodiments a combination of the above described actions can be performed. The advantage of using the combined sensor over the current situation is in reducing false alarms and improving the acceptance of the device by the user. Reports suggests that some of the subjects do not use the IGS system at nights due to the high level of false alarms.

In some embodiments the ABGS is initiated at a certain time of the day or night in anticipation of glucose changes for example around dawn where changes in glucose level are expected due to the release of certain hormones in the middle of the night which work around the action of insulin. This may lead to increase in glucose level in the early morning. To improve accuracy of the sensor at those times where large change in glucose may occur a pre-determined ABGS initiation may be applied. Also the point in time which the ABGS is initiated may be calibrated to suit individual glucose profile either during the day or during the night. Using this predetermined initiation an increased accuracy of glucose measurement may be achieved. Another phenomena which may cause temporal change in glucose is Rebound hyperglycemia which may cause a low glucose level around 3 A.M. A pre-determined initiation of the ABGS will ensure accurate glucose measurement at this point in time. The exact time of initiation may vary between individual and hence may be set individually

In some embodiments, the insulin dose or rate may be determined by a processing unit that preferably obtains the glucose level readings and most preferably derives the required insulin dose or rate that most preferably may then be infused. In some embodiments this information may be optionally and preferably displayed to a user preferably allowing the user to confirm the reading prior to infusion of the insulin. In an optional embodiments the user confirmation is not required, wherein the glucose readings are most preferably sufficiently accurate and reliable to support automatic dosing of insulin or a closed loop control system to regulate the glucose level in the body. Currently the IGS readings which are available for such closed loop system are not accurate and reliable enough for supporting that. The combination of the IGS with ABGS as described by the present application can provide the required accuracy and reliability that can safely enough support such a closed loop system. In some embodiments of the present invention a closed loop for controlling blood glucose level can be constructed by combining an insulin pump, a processing unit, an ISG sensor along with ABGS. Based on the accurate glucose measurement of the combined glucose sensor, the processing unit can calculate the required amount of insulin whether as a bolus or change to the basal rate and inject the required insulin using the insulin pump. Alternatively the processing unit will calculate the required insulin and will wait for user authorization before injection by the pump.

In some embodiments, the automatic blood glucose sampling can be done by automatically pricking the user skin in a blood rich area such as the forearm and then a applying the small blood droplet to a glucose sensor, such as a finger stick that measure the glucose level. In some embodiments the lancet using for the skin pricking is disposable and used once. In some embodiments a magazine or cartridge of lancets are used for single pricking each. In some embodiments the glucose sensor, such as finger stick used for measuring the glucose level is disposable and used once. In some embodiments a magazine or cartridge of glucose sensors is used for single glucose tests in the same device. Devices like described above are known in the art, such as described in US7041068 and many others.

The ABGS can be disposed on the forearm or the arm leg or other body parts with relatively high perfusion.

In some embodiments the ABGS includes a method for tissue stimulation to improve local blood perfusion and get a more accurate blood glucose reading. An example for local tissue stimulation that improves the local blood perfusion is heating to in intermediate temperature, such as in the range of 35-42°C, which is known in the art to improve local blood perfusion. Other types of tissue stimulations for improving local blood perfusion are optical radiation, mechanical vibrations, suction, massaging, acoustic stimulation (such as in the ultrasonic range), electrical stimulation, microwave or radiofrequency radiation, etc.

In some embodiments the suggested combination of the IGS with ABGS is used for conducting an insulin bolus estimation calculation. In some embodiments, the combination of the IGS with ABGS device communicates a bolus amount resulting from the bolus estimation calculation to the insulin pump, which delivers a bolus of insulin to a user based on the communication from the combination of the IGS with ABGS device. The insulin infusion device delivers the insulin automatically after receiving the communication from the combination of the IGS with ABGS device.

In some embodiments the ABGS and IGS may be on the same unit and at the same body location where the IGS system measures the glucose level in the ISF fluid and the ABGS measures the glucose in the blood, such as in the dermal layer which is rich in blood vessels.

In some embodiments, the combination of the IGS with ABGS device comprises a processor and an IGS sensor coupled to the processor through wired or wireless communication channel. The processor may include a display and user interface options such as buttons. The blood glucose sensor that measures the sampled blood droplets is coupled to the same processor unit through wired or wireless communication channel or to a separate processor unit which can communicate by wires or wirelessly to the IGS processor. In some embodiments both the IGS and the ABGS include processors which communicate by wires or wirelessly to a separate process to exchange information. In some embodiments both the IGS and the ABGS include processors which communicate by wires or wirelessly to each other to exchange information. In some embodiments both the IGS and the ABGS include processors which communicate by wires or wirelessly to each other to exchange information and the display unit and user interface are placed on one of the units.

In some embodiments, the combination of the IGS with ABGS device comprises a processor and an IGS sensor coupled to the processor through wired or wireless communication channel and blood glucose sensor that measures the sampled blood droplets coupled to the processor unit through wired or wireless communication channel, both combined to provide an estimation of the concentration of the glucose in the user's blood. In some embodiments a communication circuit is coupled to the processor. The processor is adapted to calculate an amount of the insulin or fluid to be infused into the user's body based upon the sensors output signal, and to cause the monitoring device communication circuit to transmit a set of data indicative of the amount of the insulin to be infused.

In some embodiments, an indicator is coupled to the combination of the IGS with ABGS device processor and adapted to provide a notification of the following events: the measuring of the output signal produced by the sensor, the calculating of the amount of the fluid, and the transmitting of the set of data by the first communication circuit.

In some embodiments the insulin infusion device comprises a processor and a drive mechanism coupled to the processor and adapted to infuse the fluid into the user's body. A communication circuit is coupled to the processor and adapted to receive the set of data from the monitoring device communication circuit. The processor is adapted to cause the drive mechanism to automatically infuse the fluid into the user in accordance with the set of data.

In some embodiments, the combination of the IGS with ABGS device communication circuit is a transmitter or a transceiver, and the infusion device communication circuit is a receiver or a transceiver.

In some embodiments, the combination of the IGS with ABGS device further comprises a user input device for inputting commands. The monitoring device communication circuit transmits the first set of data in response to a command from the input device.

In some embodiments, the combination of the IGS with ABGS device processor is further adapted to determine according to a specific algorithm, such as described before, the optimal periods for initiating the automatic blood glucose sampling to recalibrate the IGS reading. In some embodiments, the combination of the IGS with ABGS device processor is further adapted to determine according to a specific algorithm, such as described before, the optimal periods for initiating the automatic blood glucose sampling to accurately and reliably estimate the amount of insulin bolus to be infused. In some embodiments, the combination of the IGS with ABGS device processor is further adapted to determine according to a specific algorithm, such as described before, to update the insulin basal rate if required, such as in case of hypoglycemia event.

In some embodiments, the combination of the IGS with ABGS device further comprises an indicator coupled to the monitoring device processor and adapted to provide a display of the amount of the fluid and a user input device for inputting commands. The combination of the IGS with ABGS device processor is further adapted to cause the monitoring device communication circuit to transmit the first set of data in response to a first command from the input device.

It should be noted that the methods and devices described in the present application for blood glucose level estimation can be used for accurately monitor the level of other substances in the blood or the human body. The methods described for controlling an insulin infusion device can be used similarly to control infusion of other substances required to control or regulate the glucose level in the body or control or regulate other processes or substances in the human body.

Unless otherwise defined the various embodiment may be provided to an end user in a plurality of formats, platforms, and may be outputted to at least one of a computer readable memory, a computer display device, a printout, a computer on a network or a user.

Unless otherwise defined, all technical and scientific terms used herein have the same meaning as commonly understood by one of ordinary skill in the art to which this invention belongs. The materials, methods, and examples provided herein are illustrative only and not intended to be limiting. Implementation of the method and system involves performing or completing certain selected tasks or steps manually, automatically, or a combination thereof. Moreover, according to actual instrumentation and equipment of preferred embodiments of the method and system, several selected steps could be implemented by hardware or by software on any operating system of any firmware or a combination thereof. For example, as hardware, selected steps could be implemented as a chip or a circuit. As software, selected steps could be implemented as a plurality of software instructions being executed by a computer using any suitable operating system. In any case, selected steps of the method and system could be described as being performed by a data processor, such as a computing platform for executing a plurality of instructions.

It should be noted that optionally any device featuring a data processor and/or the ability to execute one or more instructions may be described as a computer, including but not limited to a PC (personal computer), a server, a minicomputer, a cellular telephone, a smart phone, a PDA (personal data assistant), a pager. Any two or more of such devices in communication with each other, and/or any computer in communication with any other computer, may optionally comprise a "computer network".

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in order to provide what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIG 1A-D provides various views of a schematic diagram of a button shaped analyte monitoring device according to an optional embodiment that may be coupled to any skin surface.
FIG. 2-4 depicts schematic diagram optional embodiment of an analyte monitoring device according to a preferred embodiment
FIG. 5A-I provide various views of schematic diagrams of an analyte monitoring device according to an optional embodiment that is adapted for use on a finger.
FIG. 6A-C provide various views of schematic diagrams of a ring shaped analyte monitoring device according to an optional embodiment that is adapted for use on a finger.
FIG 7 A-B depicts an optional embodiment of a cylindrically shaped analyte monitoring device.
FIG 8A-H visually depicts an exemplary method for sampling and measuring a bodily fluid sample with the analyte monitoring device of FIG. 1 having a stationary analyte measuring medium/element.
FIG. 9A-D visually depicts a method for sampling a bodily fluid sample with the analyte monitoring device of FIG. 1 equipped with a movable analyte measuring medium/element
FIG 10 shows a flowchart of an exemplary method.
FIG. 11A-C provide a schematic block diagram of a closed loop analyte monitoring system.
FIG. 12 A-D show optional modular devices that can associate within the optional embodiments of the analyte monitoring devices .
FIG 13 shows a flowchart of an exemplary method for closed loop analyte monitoring system and device.
FIG 14A-C depicts accessories to the optional embodiments of the modular analyte measuring device of Figures 1-7.
FIG. 15A-B depicts an optional analyte measuring medium according to an optional embodiment.
FIG. 16 depicts an optional system for IGS in use with an analyte measuring medium according to an optional embodiment .

### DESCRIPTION OF THE PREFERRED EMBODIMENTS

The present invention relates to an analyte measuring system according to claim 1.

Preferably the device provides for measuring at least one or more analytes in an automatic predetermined manner while optionally and preferably inferring an action based on such monitoring. Optionally the device may also be linked to a plurality of devices to form a closed loop system.

The principles and operation may be better understood with reference to the drawings and the accompanying description.

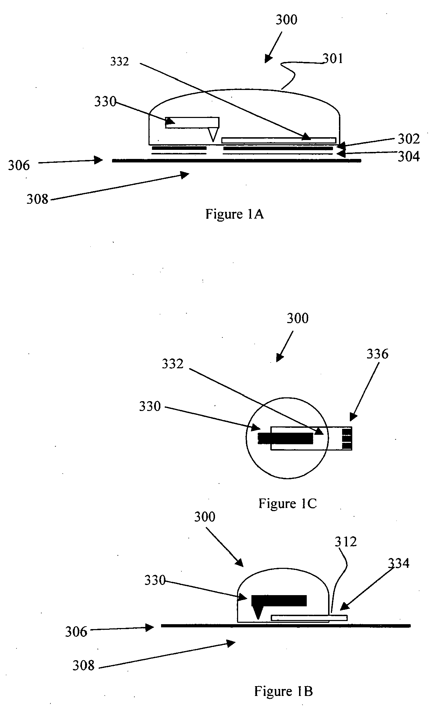

Figures 1A-D depict optional embodiments of an analyte monitoring device 300. Most preferably analyte monitoring device 300 comprises a housing 301 that may optionally assume various geometric shapes for example including ellipsoid, circular, square, button, disc, or the like geometric shape. Preferably analyte monitoring device 300 comprises at least one or more piercing element 330 and at least one or more analytes measuring medium 332.

Most preferably, device 300 may be securely attached to or coupled to any external skin surfaces 306 that would allow piercing element 330 to extract or draw sufficient bodily fluid, for example including but not limited to blood and/or interstitial tissue, from an underlying tissue 308 for analyte measurement and analysis. Optionally device 300 may be adhered, attached or otherwise coupled to an external skin surface for example including but not limited to hip, back, abdomen, arm, leg or the like skin surface. Preferably device 300 is preferably be attached to body locations used as primary or alternate sites for capillary glucose measurements such as the arm or leg.

Optionally and preferably, device 300 may be provided with an adhesive layer 304 to facilitate the coupling of device 300 to skin surface 306 underlying tissue 308.

Optionally, device 300 may comprise at least one or more tissue treatment element 304 for improving analyte measurement and/or analysis. Optionally, at least one or more tissue treatment element may optionally be use for example including but not limited to electrical energy, ultrasound energy, optical energy, acoustic energy, vasodilatation drugs or the like. For example, a heating pad may be disposed on the lower surface of device 300 between adhesive layer 304 and bottom surface of housing 301.

Optionally housing 301 may be adapted to accepted interchangeable faces for example including a decorative faces.

Most preferably, device 300 may be activated remotely via communication protocols known and accepted in the art for example including but not limited to contactless, RF, wireless, cellular, IR or the like communication protocols.

Most preferably, device 300 may further comprise a communication module (not shown) providing for two way communication, for example both receiving data and sending data using communication protocols known and accepted in the art for example including but not limited to contact-less, RF, wireless, cellular, IR or the like communication protocols.

Optionally and preferably, analyte measuring device 300 may comprise a single device that may optionally be associated with disposable or interchangeable analyte measuring mediums 332 while its functional parts maintained in a single housing. Optionally, disposable portion may comprise piercing element 330. Optionally disposable portion may be composed of at least one of analyte measuring medium 332 and/or piercing element 330.

Most preferably, analyte monitoring device 300 comprises at least two or more portions functionally separable portions, wherein a first portion is a reusable portion (not shown) that is configured to be reusable allowing for repeated use while a second portion (not shown) configured to be disposable optionally for a limited time and/or single use. Optionally, disposable portion and reusable portion may be readily and securely associated or disassociated with one another. Optionally and preferably the secure association or disassociated is facilitated by the use of connectors that may be functionally manipulated to associate or disassociate reusable portion with disposable portion.

Optionally and preferably, device 300 is realized as a modular analyte monitoring device that may be a member of a greater system for closed loop monitoring of an analyte sampled from a bodily fluid. Optionally and preferably the continuous monitoring system comprises active control of the three primary monitoring facets including continuous monitoring, continuous response - optionally drug response- and continuous centralized processing and control. Optionally, device 300 can directly interface, sync and link with a central controller device. Optionally, device 300 can interface and interact with a plurality of devices within the closed loop system. Optionally, a plurality of modular devices 300 may interface with one another optionally using a communication port.

Figure 1A provides a side view of device 300 comprising housing 301, at least one or more piercing element 330, at least one or more analytes measuring medium 332, an adhesive layer 304, and tissue treatment element 302. Tissue treatment element comprises a heating pad disposed on the lower surface of housing 301 providing heating to the underlying skin surface 306 and tissue 308, preferably for to improve blood perfusion. Adhesive layer 304 provides for coupling housing 301 to skin surface 306.

Most preferably, piercing element 330 protrudes and pierces the underlying skin layer 306 through opening 310. Most preferably, opening 310 allows for bodily fluid to flow from the underlying tissue 308 into opening 310 wherein it may be reacted with at least one or more analytes measuring element 332.

Figure IB provides a schematic depiction of an analyte monitoring device 300 configured around an analyte measuring element similar to standard glucose finger stick 334. Device 300 is manufactured around analyte measuring element 334 to includes all disposable components required for a single analyte measurement. In another optional embodiment similar to the device shown at Figure IB, device 300 is configured to receive an external measuring element 334 through a second opening 312 on the external surface of housing 301 for accepting an external analyte measuring element 334. Figure 1C provides a top down view of device 300 of Figure IB wherein external analyte measuring element 334 is optionally provided externally through opening 312. Analyte measuring element 334 comprises at least one or more preferably a plurality of external electrical contacts to provide for measuring the analyte in a bodily fluid.

Figure ID provides yet another optional depiction of analyte monitoring device 300 further comprising a movement module 350 for example including but not limited at least one or more of a gear works assembly, mechanical rotary assembly, electrical rotary assembly, acoustic rotary assembly, piezoelectric rotary assembly or the like. Most preferably movement module 350 is associated with at least one or more analytes measuring element 332 to allow it to displace from an initial position to a position that is in contact with a bodily fluid accumulating over opening 310 and optionally to a final position. Optionally, movement module 350 may be utilized to displace at least two or more analytes measuring elements 332.

Optionally the directional displacement of measuring element 332 by movement module 350 may be rotational providing for up to 360 degree movement form an initial position within housing 301. Optionally rotational motion may be from about 20 degree movements to 180 degree displacement form an initial position within housing 301. In general, rotational motion fits more to analyte measuring element 332 which has side opening for sampling of bodily fluid.

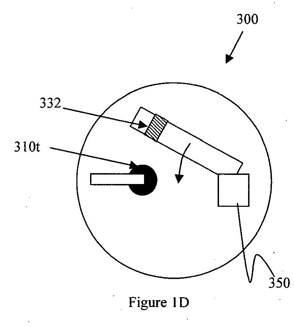

Optionally directional displacement of measuring element 332 by movement module 350 may optionally be planar providing for displacement in the horizontal plane and or vertical plane. Optionally, directional displacement of measuring element 332 by movement module 350 may optionally comprise both planar and rotational movements. Optionally, directional displacement of measuring element 332 by movement module 350 may optionally be arched with respect to lower surface of housing 301. Optionally directional displacement of measuring element 332 by movement module 350 may to displace measuring element 332 from an initial position outside of housing 301 for example a position defined by opening 312 as described in Figure 1D. Optionally, directional displacement of measuring element 332 by movement module 350 may in a plane perpendicular to the plane of the measuring surface for example allowing for the up and down movement of at least one or more measuring element 332, for example along the shaft of piercing element 330.

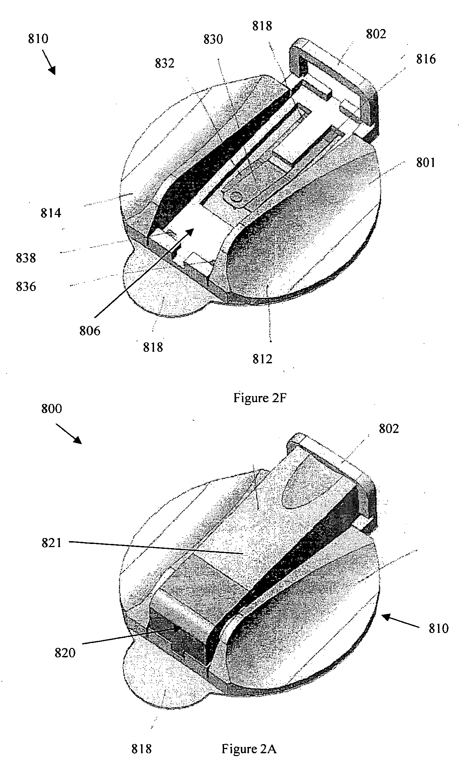

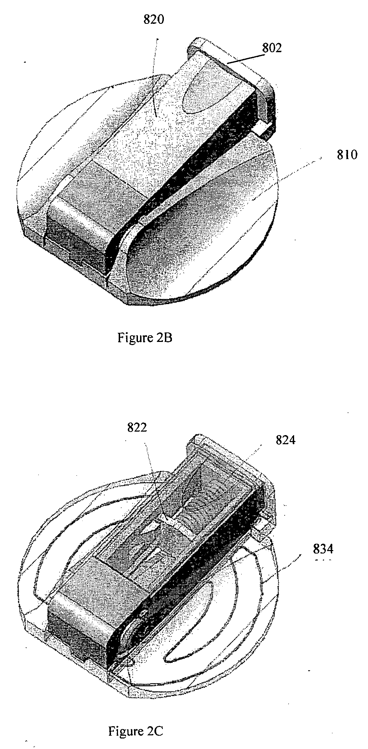

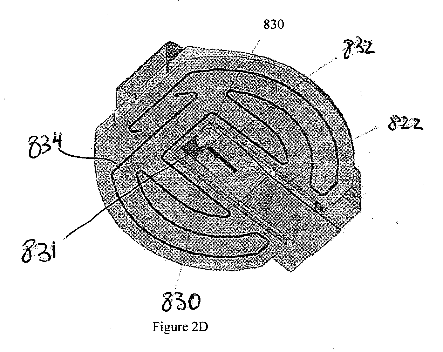

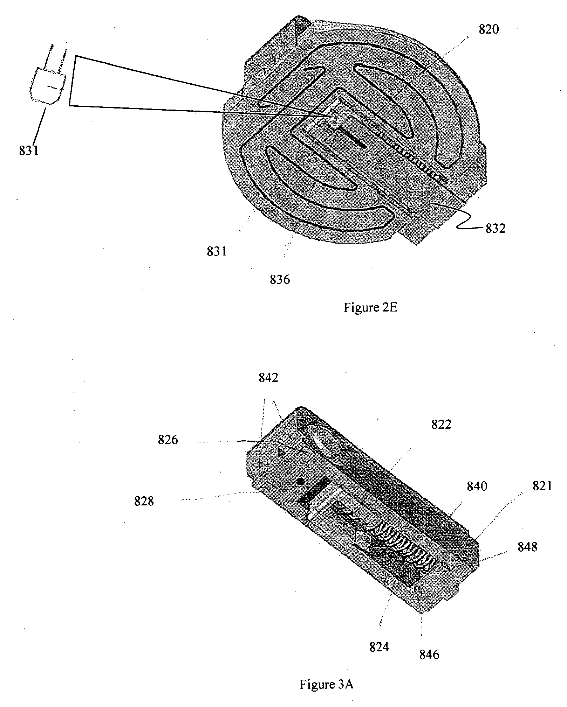

An optional embodiment of analyte measuring device 300 of Figure 1A-C is depicted in Figures 2-4, where analyte measuring device 300 is adapted to form analyte measuring device 800 comprising at least two or more portions wherein a first portion is configured to be reusable allowing for repeated use while a second portion is configured to be disposable optionally and preferably for limited and/or single time use.

Most preferably, analyte monitoring device 800 comprises at least two or more portions wherein a first portion 820, also referred as reusable portion, is configured to be reusable allowing for repeated use while a second portion 810 is configured to be disposable optionally for limited and/or single time use. Most preferably disposable portion 810 and reusable portion 820 may be readily and securely associated or disassociated with one another. Optionally and preferably the secure association or disassociated is facilitated by the use of connectors that may be functionally manipulated to associate or disassociate reusable portion 820 with disposable portion 810.

Most preferably, disposable portion comprises disposable elements and parts that are adept for single or limited use that are preferably associated with obtaining and/or sampling a bodily fluid. Most preferably reusable portion 820 is configured to provide at least one or more state of the art sensors or the like for measuring the sampled bodily fluid, for example including but not limited to amperemetric, acoustic, ultrasonic, optical, electromagnetic, infrared or the like systems or methods for measuring an analyte from a bodily fluid as is known and accepted in the art.

Most preferably, disposable portion 810 comprises: housing 801, a recess 806 for receiving and connecting with reusable portion 820, at least one or more piercing elements 830, clamp 802 for coupling to reusable portion 820, an analyte analysis element 832, electrical contacts 816 and 818 for analyte analysis element, association electrical contact connectors 838 and 836, two wings 812 and 814 preferably disposed on either side of recess 806 preferably for extracting bodily fluid for analysis by squeezing the pierced tissue; a heating element 834, and coated with biocompatible adhesive layer coating 816 on its bottom side covered with laminate 818.

Most preferably, the reusable portion 820 comprises housing 801, piston 822, spring 824, at least two or more electrical contact couplers 846 and 848, electrical contacts 826 and 828, at least one or more coupling recess 842 and controller 840.

Optionally and preferably, device 800 may be provided with an adhesive layer 816 to facilitate the coupling of device 800 to skin surface where measurement is to be undertaken.

Optionally housing 801 and/or housing 801 may be adapted to accepted interchangeable faces for example including decorative faces.

Most preferably, device 800 may be activated remotely via communication protocols known and accepted in the art for example including but not limited to contactless, RF, wireless, cellular, IR or the like communication technology and/or protocols.

Most preferably, device 800 may further comprise a communication module (not shown) providing for two way communication, for example both receiving data and sending data using communication protocols known and accepted in the art for example including but not limited to contact-less, RF, wireless, cellular, IR or the like communication protocols.

Optionally, device 800 may comprise at least one or more tissue treatment elements for improving analyte measurement and/or analysis. Optionally, at least one or more tissue treatment element may optionally be used for example including but not limited to heating, massaging, mechanical stimulation, electrical energy, ultrasound energy, optical energy, acoustic energy, vasodilatation drugs or the like.

Optionally and preferably, device 800 is realized as a modular analyte monitoring device that may be a member of a greater system for closed loop monitoring of an analyte sampled from a bodily fluid. Optionally and preferably the continuous monitoring system comprises active control of the three primary monitoring facets including continuous monitoring, continuous response - optionally drug response- and continuous centralized processing and control. Optionally, device 800 can directly interface, sync and link with a central controller device. Optionally, device 800 can interface and interact with a plurality of devices within the closed loop system. Optionally, a plurality of modular devices 800 may interface with one another optionally using a communication port.

Figure 2A provides a prospective view of schematic depiction of analyte measuring device 800 according to the present invention comprising disposable portion 810 coupled to reusable portion 820 with clip 802. Device 800 may be attached to the skin over the targeted measurement area using adhesive layer 816, covered by a laminate 818. Figure 2B provides the same view as Figure 2A where laminate 818 has been removed from device 800 in preparation for use, attachment to the skin surface.

Figure 2C provides a see through view of disposable body 801 and reusable body 821 revealing the respective inner workings of disposable portion 810 and reusable portion 820. Figure 2C reveals the tissue treatment element 834 disposed on the underside of disposable portion 810, optionally and preferably is provided in the form of a heating element that is attached to the skin preferably promote blood perfusion and improve analyte measurement properties. Figure 2C further reveals piston 822 and spring 824 disposed within reusable portion 820. Preferably piston 822 and spring 824 is used to facilitate the piercing element 830 (not shown) when sampling a bodily fluid.

Figure 2D and 2E provides a bottom up view of device 800 in different states of functionality. Figure 2D depicts the state of the piercing element prior to deployment wherein lancet 831 has not reach the skin surface (not shown) to obtain a sample of bodily fluid, for example including but not limited to blood. Most preferably piston 822 is utilized to trigger the movement of piercing element 830 and lancet 831 from the position depicted in Figure 2D to the position 2E. Preferably and optionally piston 822 is triggered by a controller 840 (not shown) that optionally functions according to a schedule for example time of day, postprandial, and/or a communicated protocol according to an optional embodiment of the present invention. Optionally prior to sampling the bodily fluid, tissue treatment element 834 is activated by controller 840 according to an optional treatment protocol. For example, approximately 5 minutes prior to measurement heating element 834 is activated to heat the tissue measuring sit to a comfortable temperature for about 5 minutes, optionally heating element 834 heat the sampling tissue (not shown) to a temperature in the range of 37-43 degrees Celsius. Preferably, such heating protocol increases local blood perfusion, increases the blood volume in the sampling tissue region (not shown) and enable easier and better withdrawal of bodily fluids for example blood when piercing the sampled tissue region. At the required measurement time piston 822 is released and pushed by spring 824 forward, pushing piercing element 830 with lancet 831 toward the skin surface and piercing it to release a bodily fluid. Piston 822 stay at the end position of its path as shown at Figure 2E. Most preferably in this position the lancet 831 and piercing element 830 are ejected from the skin surface by the force of a flexible beam within piercing element 830 Figure 2E further shows that analyte measuring element 832 remains extended to allow for the analyte measuring element 832 to sample the bodily fluid within its measurement channel 836. Most preferably piercing element 830 comprising lancet 831 having a sharp, blade-like scoring edge (see close up view) that is preferably about 1.2mm wide and is adept at piercing a tissue within the extremities or limbs, also referred to as alternate sites preferably to provide sufficient sample size for measuring blood glucose levels from an arm and or leg, for example.

Most preferably lancet 831 may be provided in optional different shapes for example including but not limited to lancet, blade, blade edge, sharp edge, needle, sharp point, scoring edge, tapered edge, cross shaped blade or the like depending on where the measurement is to be taken and the blood volume required for the analyte measurement by an analyte measuring element. For example, lancet 831 is most preferably provided in the shape of a scoring blade-like edge when measurement is performed at an alternative piercing site, for example including but not limited to a leg and/or arm. A blade like lancet 831 preferably increases the likelihood of producing a piercing that provides sufficient blood volume to allow for blood glucose measurement with an analyte measuring element, most preferably a finger stick, when used at an alternate measuring site for example a leg and/or arm, or the like measuring site, with a minimum discomfort to the user. For example when measuring from the finger tip lancet is most preferably provided in the form of a sharp pointed edge or needle.

Measurement is undertaken as described above and known in the art.

Optionally and preferably reusable portion 820 and disposable portion 810 are separated wherein a disposable portion 810 is disposed of while a new one is attached to the same reusable portion 820.

Figure 2F depicts the disposable unit 810 in its independent or separated form comprising: housing 801, a recess 806 for receiving and connecting with reusable portion 820, for at least one or more piercing elements 830, clamp 802 for coupling to reusable portion 820, an analyte analysis element 832, electrical contacts 816 and 818 for analyte analysis element, association electrical contact connectors 838 and 836, two wings 812 and 814 preferably disposed on either side of recess 806 preferably for manually assisting the extraction of a bodily fluid by squeezing the pierced tissue; a heating element 834, and coated with biocompatible adhesive layer coating 816 on its bottom side covered with laminate 818.

In an optional embodiment after piercing, the controller initiates a predetermined time lag, for example about 7 seconds, before moving the analyte measuring element toward the released blood droplet. Preferably such a time lag provides sufficient time for bloodletting and pooling at the piercing site to accumulate a sufficient blood volume. Preferably and optionally, a time lag may be coordinated with the type of lancet used with the piercing element. For example, using a blade like lancet 831 combined delay of 7 sec before measuring the blood sample provides increased likelihood for extracting a large enough blood sample from the measuring site, most preferably an alternate site such as a limb, arm and/or leg. For example, this optional combination could produce a blood sample of about 0.6 micro liters of blood, which is a preferred for volume for an analyte measuring element, for example glucose test strips. Optionally, other analyte measuring elements prefer a blood sample size volume in the range of about 0.3 to 1 micro liter, where the 0.3 micro liter test strips are the most preferably for measuring the blood glucose level at alternate sites, such as limbs, arm or leg. In some embodiments to further increase the likelihood of obtaining a sufficiently large blood sample a specific type of treatment element may be applied to the measurement are before and/or after piercing. For example, massaging the piercing site preferably using wings 812 and 814 or optionally heating the tissue, preferably using heating element 834 in and around the piercing site, to preferably increase the local blood perfusion. Optionally other optional treatment elements may be used and coordinated with the time delay before measuring the blood sample with analyte measuring element, such as the glucose test strip.

Most preferably a time lag may be adapted to allow sufficient blood volume to accumulate preferably according to at least one or more parameters, for example including but not limited to measurement location, lancet type, local blood perfusion, treatment element and the employed analyte measuring element or an analyte measuring sensor..

In some embodiment a specific blood volume sensor, for example an optical sensing element may be provided to verify that large enough blood sample has been extracted. Optionally, such a blood volume sensor may be utilized by the controller to coordinate movement of the analyte measuring element toward the blood sample most preferably to perform the analyte measurement with increased likelihood of obtaining a successful and accurate measurement of the blood glucose level. Optionally a blood glucose volume sensor may be incorporated into analyte measuring element, such as the glucose test strip. Optionally a blood volume sensor may be disposed within the housing of the analyte measuring device , for example within the disposable portion and/or reusable portion. Figure 3A-C provides view of the reusable portion 820 alone showing different states of piercing element and the driving piston 822. Figure 3A depicts reusable portion 820 following disassociation from disposable portion 820 (not shown). The bottom up view reveals electrical connectors 846 and 848 preferably connect to the analyte measurement element 832. Preferably, electrical contacts 826 and 828 connect to heating element 834 electrical contacts when disposable portion 810 is associated with and coupled to reusable portion 820. Preferably, at least two or more recesses 842 provide over the corresponding connector of disposable portion 810. Reusable portion 810 comprise a controller 840. Optionally and preferably controller 840 is used to facilitate two way communication between device 800 and other devices associated therewith.

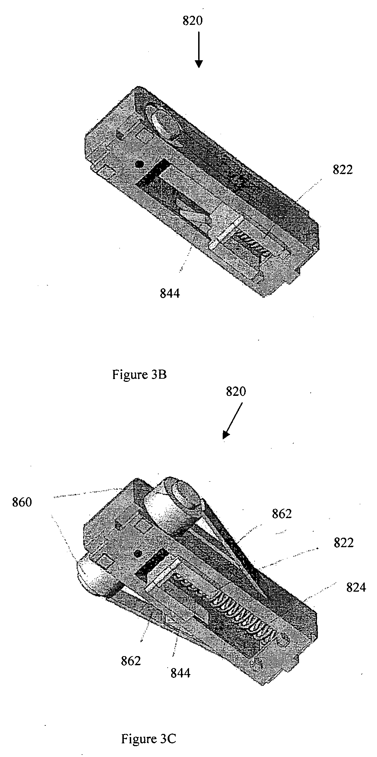

Figure 3B provides a schematically diagram of reusable portion 810 prior to attachment to a new disposable portion 820 (Figure 2F). To associate reusable unit 820 with a disposable unit, the piston 822 is retracted and locked in its starting position and is preferably locked with lock connector 844.

Figure 3C provides a depiction of the inner working of the reusable portion 820 when piston 822 is activated through controller 840 where piston 860 are extended , which in turn push at least two or more leaf springs 862 to release driving piston 822 by releasing lock connector 844 to trigger the piston 822 and spring 824 accordingly as previously described.

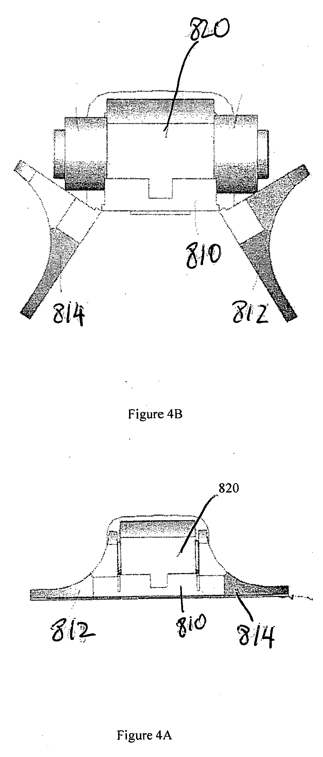

Figure 4A-B depicts the mechanical movement of device 800 wherein disposable portion 810 is squeezed over the sampling tissue area by releasing wings 812 and 814 and rotating them about their access. Optionally, mechanical movements and/or massage about the piercing site improves the likelihood of releasing sufficient amount of bodily fluids most preferably blood. Preferably mechanical movement are provided with the up and down movement in and around the piercing site with a piston (not shown) that massages the tissue around the piercing point.

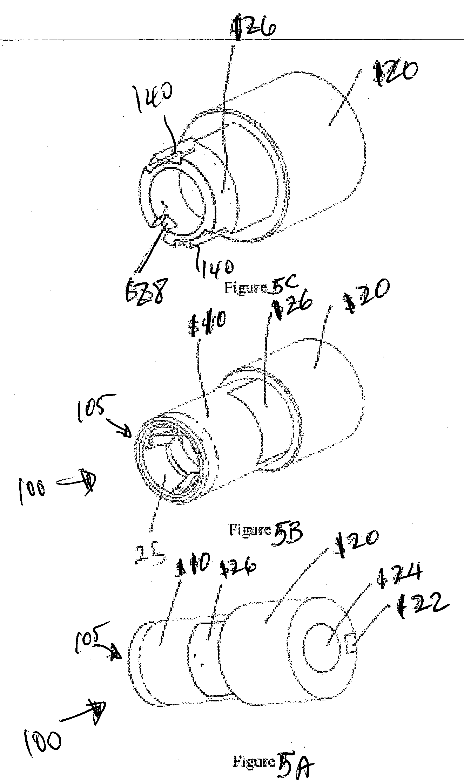

Figure 5A-I provides varying views and cutaway section of an optional embodiment for an analyte monitoring device 100 that is preferably configured for use on extremities for example including but not limited to a hand or foot and most preferably adapted for use on a finger or toe. Optionally, analyte measuring device 100 may comprise a single device that may optionally be associated with disposable analyte measuring mediums while its functional parts maintained in a single housing.

Most preferably, analyte monitoring device 100 comprises at least two or more portions wherein a first portion 120, also referred as reusable portion, is configured to be reusable allowing for repeated use while a second disposable portion 110 is configured to be disposable optionally for a limited and/or single time use. Most preferably disposable portion 110 and reusable portion 120 may be readily and securely associated or disassociated with one another. Optionally and preferably the secure association or disassociated is facilitated by the use of connectors that may be functionally manipulated to associate or disassociate reusable portion 120 with disposable portion 110.

Most preferably, disposable portion comprises disposable elements and parts that are adept for single or limited use that are preferably associated with obtaining and/or sampling a bodily fluid. Most preferably reusable portion 120 is configured to provide at least one or more state of the art sensors or the like for measuring the sampled bodily fluid, for example including but not limited to amperemetric, acoustic, ultrasonic, optical, electromagnetic, infrared or the like systems or methods for measuring an analyte from a bodily fluid as is known and accepted in the art.

Most preferably, disposable portion 110 comprises: housing 101, at least one or more connectors 107, at least one or more piercing elements 130, rotational element 103 and extremities adaptor element 105.

Most preferably, the reusable portion 120 comprises housing 102, rotating element 126, at least one or more analytes monitoring and measuring elements 146 and 148, display indicator 122 and electronic module 124, which comprises processing unit, power source and communication element.

Optionally housing 102 and/or housing 101 may be adapted to accepted interchangeable faces for example including decorative faces.

Most preferably, device 100 may be activated remotely via communication protocols known and accepted in the art for example including but not limited to contactless, RF, wireless, cellular, IR or the like communication protocols.

Most preferably, device 100 may further comprise a communication module (124)providing for two way communication, for example both receiving data and sending data using communication protocols known and accepted in the art for example including but not limited to contact-less, RF, wireless, cellular, IR or the like communication protocols.

Optionally, device 100 may comprise at least one or more tissue treatment elements for improving analyte measurement and/or analysis. Optionally, at least one or more tissue treatment element may optionally be used for example including but not limited to heating, mechanical stimulation, electrical energy, ultrasound energy, optical energy, acoustic energy, vasodilatation drugs or the like.

Optionally and preferably, device 100 is realized as a modular analyte monitoring device that may be a member of a greater system for closed loop monitoring of an analyte sampled from a bodily fluid. Optionally and preferably the continuous monitoring system comprises active control of the three primary monitoring facets including continuous monitoring, continuous response - optionally drug response- and continuous centralized processing and control. Optionally, device 100 can directly interface, sync and link with a central controller device. Optionally, device 100 can interface and interact with a plurality of devices within the closed loop system. Optionally, a plurality of modular devices 100 may interface with one another optionally using a communication port.

Figure 5A provides a perspective view of analyte monitoring device 100 depicting both reusable portion 120 and disposable portion 110 while both are associated with one another. Figure 5A provides a view of the external face of reusable portion 120 depicting indicator 122 for example in the form of a display, LED, LED array, LCD or the like display and/or indicator.

Optionally and preferably, rotating element 126 provides an interface between reusable portion 120 and disposable portion 110. Preferably rotating element 126 functions to trigger the corresponding rotation elements 103 and piercing element 130 (not shown here) disposed in disposable portion 110.

Most preferably, the rotating interface provided by rotating element 126 allows for the rotation of disposable portion 110 relative to the reusable portion. Optionally rotating element may be activated manually, remotely or automatically. Optionally rotating element may be activated according to a schedule. Optionally, rotating element 126 may be activated remotely via communication protocols known and accepted in the art for example including but not limited to wireless, cellular, IR or the like communication protocols.

Figure 5B provides a perspective view of analyte monitoring device 100 depicting both reusable portion 120 while associated with disposable portion 110. Figure 5B provides a further view of the external face of disposable portion 110 revealing the extremities adaptor element105.

Optionally and preferably extremities adaptor element 105 comprises finger adaptor 150 (not shown) and at least one or more preferably a plurality of fine tuning extremities adjusting elements 152. Most preferably extremities adjusting elements 152 provide for a comfortable and secure fit for analyte measuring device 100 over an extremity for example including a finger or toe.

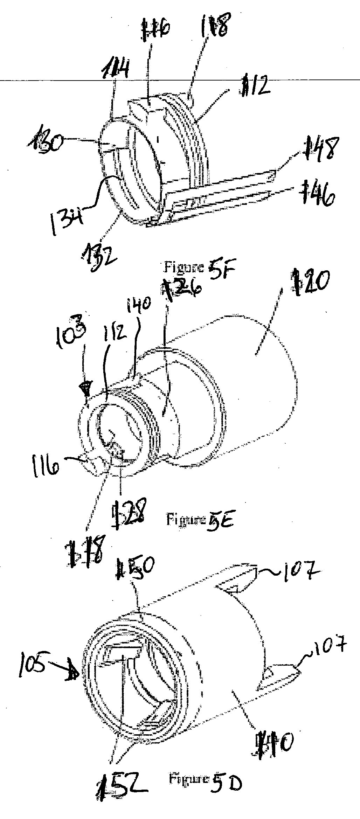

Figure 5C depicts reusable portion 120 in its disassociated state from disposable portion 110. This view depicts an optional mechanism for controllably associating and disassociating reusable portion 120 from disposable portion 110 using rotational element 126 comprising at least one and more preferably a plurality of recesses 140 adept to accepting corresponding connectors positioned on disposable portion 110, for example connectors 107 (not shown here see Figure 5D). Figure 5C further depicts the rotational interfaced disposed on rotational element 126 to trigger the rotation of disposable portion 110 through recess 128 adept to accepting and interfacing with the corresponding dowel 118 (not shown here, see Figure 5E).

Figure 5D provides a perspective view of disposable portion 110 in its disassociated state revealing the associating connectors 107 that may be associated or disassociated with the recess 140 of Figure 5C. Figure 5D further provides a closer depiction of the extremities adaptor element 105 comprises finger adaptor 150 for accepting and/or receiving an extremity while at least one and more preferably a plurality of fine tuning extremities adjusting elements 152 provide a comfortable and secure placement of analyte measuring device 100 over an extremity (not shown).

Figure 5E provides a cutaway view of reusable portion 120 that is partially disassociated with disposable portion 110. Figure 5E depicts the association between rotational element 126 and the corresponding rotational elements 103 though the association of recess 128 and dowel 118. Rotational element 103 comprises rotating ring 112 that most preferably provides for the rotation of piercing element 130 (not shown) comprised with disposable element 110. Rotational element 103 further comprises rounded beam 116 that is preferably used to associate with piercing element 130 (not shown).

Figure 5F depicts a further cutaway of Figure 5E wherein reusable housing 102 has been removed showing the associated rotational element 103 with the piercing element 130 both most preferably disposed with disposable portion 110 and showing analyte measuring element 132 which is in electrical contact with at least two electric conductors 146 and 148 (for simplicity only two wires are shown at Fig. 5F). Analyte measuring element 132 is preferably disposed within reusable portion 120 and controllably associated with corresponding electrical conductors 146 and 148 through corresponding electrical contacts 136 and 138 disposed on analyte measuring element 132 within disposable portion 110. Most preferably measuring element 132 is realized to provide for analyte measuring and analysis. Measuring element 132 may take a plurality of optional forms depending on the analysis undertaken for example including but not limited to amperemetric, acoustic, ultrasonic, optical, electromagnetic, infrared. For example, for ameperemetric analysis measuring elements 132 may be realized through as electrical contacts 136 and 138 and or conductors 146 and 148.

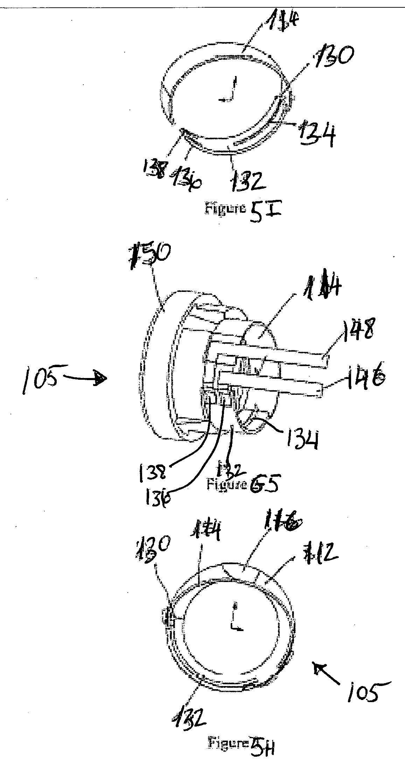

Figure 5G provides a further cutaway view depicting disposable portion 110 and measuring element 132 with electrical contacts 136 and/or 138 which are associated with at least two electrical conductors 146 and 148 which are connected on their other side (not shown) to said reusable part.

Figure 5H provides a schematic planar cutaway view of disposable portion 110 showing disposable rotation portion 103 comprising rotating ring 112 associated with rounded beam 116 that interact to trigger the bend of flexible beam 114 and movement of piercing element 130 into the skin..

Preferably, rotating element 126 rotates, for example at about 100 degrees, counterclockwise around the extremity associated thereto preferably a finger and/or toe. Preferably, the rotational angle depends on the configuration and may be adapted to a user or a particular configuration. Optionally ration may be set to be up to 360 degrees; optionally and preferably up to about 180 degrees. Most preferably rotational motion is powered by a circular spring (not shown) disposed inside reusable portion 120. Optionally and preferably, when rotating element 126 is triggered it brings about the rotation of rotating ring 112 and its rounded beam 116 comprised in disposable rotational element 103. Most preferably, rounded beam 116 is displaced counterclockwise triggering the bend of piercing element 130 via flexible beam 114, preferably causing it to pierce the skin of the extremity attached thereto. Then rotating ring 112 continues to rotate an additional 10 degrees to deactivate therein releasing piercing element 130 and flexible beam 114 while moving analyte measuring medium 132, most preferably a test strip, toward the piercing site on the extremity surface. Body fluid, for example including but not limited to blood, is expelled from the piercing site then comes into contact with analyte measuring medium 132 and/or capillary channel 134. Preferably and optionally capillary channel 134 is defined by a recess or otherwise embedded within analyte testing medium 132. Optionally and preferably, bodily fluid is the displaced within the capillary channel 134 optionally and preferably by capillary action, wherein an analyte for example including but not limited to glucose is measured.

Figure 5I provides a schematic view of an exemplary disposable piercing element 130, analyte measuring medium 132, analyte capillary channel 134, at least one or electrical contacts 138 and/or 136. Optionally and preferably capillary channel 134 is defined by a recess or otherwise embedded within analyte testing medium 132. Most preferably analyte testing medium 132 is a test strip as is known in the art. Figure 5I further depicts the interaction between a portion of disposable rotational element 103 and the piercing element 130 are associated with flexible beam 114 and rounded beam 116 to allow for rotation about the extremity.

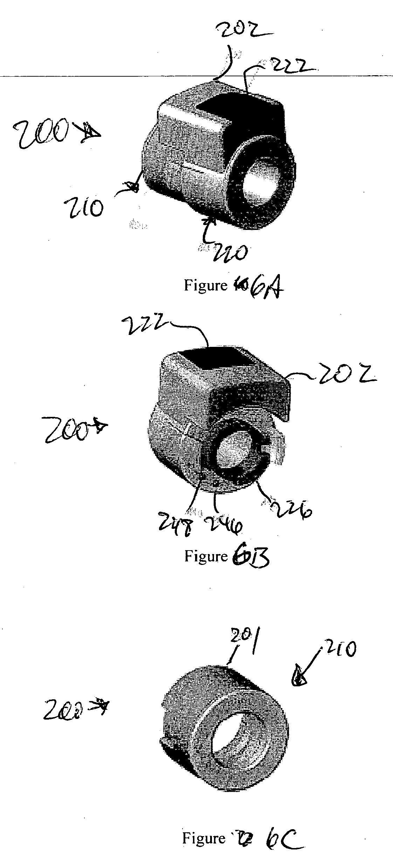

Figure 6A-C provide a further schematic diagram of an optional embodiment of the present invention for an ring shaped analyte measuring device 200 that may be placed over an extremity for example including a finger or a toe. Most preferably, ring shaped analyte measuring device 200 is similar in function to analyte measuring device described in Figure 5A-I. Most preferably, ring shaped device 200 may be composed of two associative coupled portions a disposable portion 210 comprising housing 201 and reusable portion 220 comprising housing 202. Most preferably, disposable portion 210 is similar to disposable portion 110 described before. Preferably, disposable portion 210 rotates clockwise relative to reusable portion 220, preferably utilizing a spring housed within reusable housing 202, as described in Figure 5H.

Optionally, ring shaped device 200 may comprise a single device that may optionally be associated with disposable analyte measuring mediums while its functional parts maintained in a single housing.

Optionally, device 200 may comprise at least one or more tissue treatment element (not shown) for improving analyte measurement and/or analysis. Optionally, at least one or more tissue treatment element may optionally be use for example including but not limited to heating, mechanical stimulation, electrical energy, ultrasound energy, optical energy, acoustic energy, vasodilatation drugs or the like.

Optionally housing 202 and/or housing 201 may be adapted to accepted interchangeable faces for example including decorative faces.

Most preferably, device 200 may be activated remotely via communication protocols known and accepted in the art for example including but not limited to contactless, RF, wireless, cellular, IR or the like communication protocols.

Most preferably, device 200 may further comprise a communication module (not shown) providing for two way communication, for example both receiving data and sending data using communication protocols known and accepted in the art for example including but not limited to contact-less, RF, wireless, cellular, IR or the like communication protocols.

Optionally and preferably, device 200 is realized as a modular analyte monitoring device that may be a member of a greater system for closed loop monitoring of an analyte sampled from a bodily fluid. Optionally and preferably the continuous monitoring system comprises active control of the three primary monitoring facets including continuous monitoring, continuous response - optionally drug response- and continuous centralized processing and control. Optionally, device 200 can directly interface, sync and link with a central controller device. Optionally, device 200 can interface and interact with a plurality of devices within the closed loop system. Optionally, a plurality of modular devices 200 may interface with one another optionally using a communication port.

Figure 6A provides a schematic depiction of ring shaped device 200 comprising disposable portion 210 (Figure 6C) associated with reusable portion 220 while Figure 6B depicts reusable portion 220 and Figure 6C depict the disposable portion 210.

Reusable portion 220 depicted in Figure 6B functions and comprises similarly functioning parts as that described in Figure 5A-I relating to the reusable portion 120. Most preferably, reusable portion 220 housing 202, rotating element 226, at least one or more electrical contacts 246 and 248 and indicator 222. Indicator 222 may optionally be provided in the form of a display comprising LED, LED array, LCD, touch screen or the like display and/or indicator.

Figure 6C depicts disposable portion 220 depicted that functions and comprises similarly parts as that described in Figure 5A-Irelating to the disposable portion 110, for example comprising rotational element 103, piercing elements 130, analyte measuring element 132 and extremities adaptor element 105.

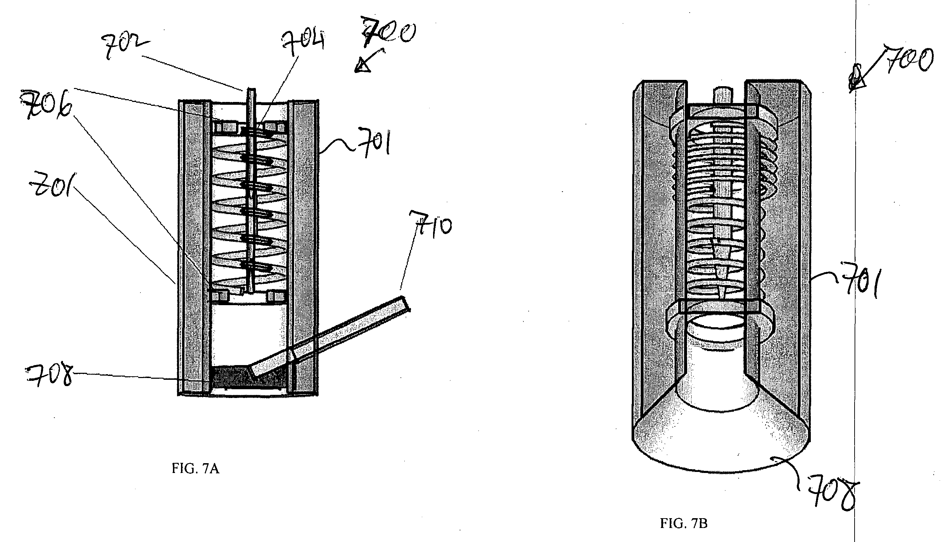

An optional embodiment of analyte measuring device 300 of Figure 1B-C is depicted in Figure 7A wherein device 300 assumes a cylindrical shape forming cylindrical analyte measuring device 700. Cylindrical analyte measuring device 700 comprises cylindrical housing 701, piercing element 702, spring 704, stoppers 706, pooling area 708, analyte measuring element 710 and side opening 712. Optionally device 700 comprises a treatment element (not shown), as described previously Figure 1, and/or an adhesive layer (not shown), as described previously Figure 1, to couple it to the skin surface.

Piercing element 702 is driven trough the center of the cylinder body 701 with spring 704 to piercing the skin surface (not shown) and than it retracts to return to its steady state position. The blood is then pooled at pooling area 708 at the bottom of hollow cylinder 701. Optionally, after a predetermined time or optionally after sufficient bodily fluid is pooled into pooling area 708 an analyte measuring element 710 is advanced into the cylinder trough a side opening 712, optionally with a movement module 350 described in detail in Figure 1B-C.

Figure 7B provides an optional depiction of analyte measuring device 700 wherein housing 701 is made of flexible material. Preferably flexible housing 701 provides for changing the inner diameter of housing 701 therein controlling the shape, size and pressure exerted at pooling area 708. For example, if inner diameter of housing 701 is expanded it will allow for increased blood volume to extracted and pooled. For example, if inner diameter of housing 701 is retracted it will allow for a reduction in blood volume and amount of pooled blood; optionally this may be used for coagulating or stopping blood flow. For example, cylinder housing 701 may be expanded by using an expandable cylinder body 701 or by inserting an element to expand the cylinder, for example including but not limited to analyte measuring element 710.

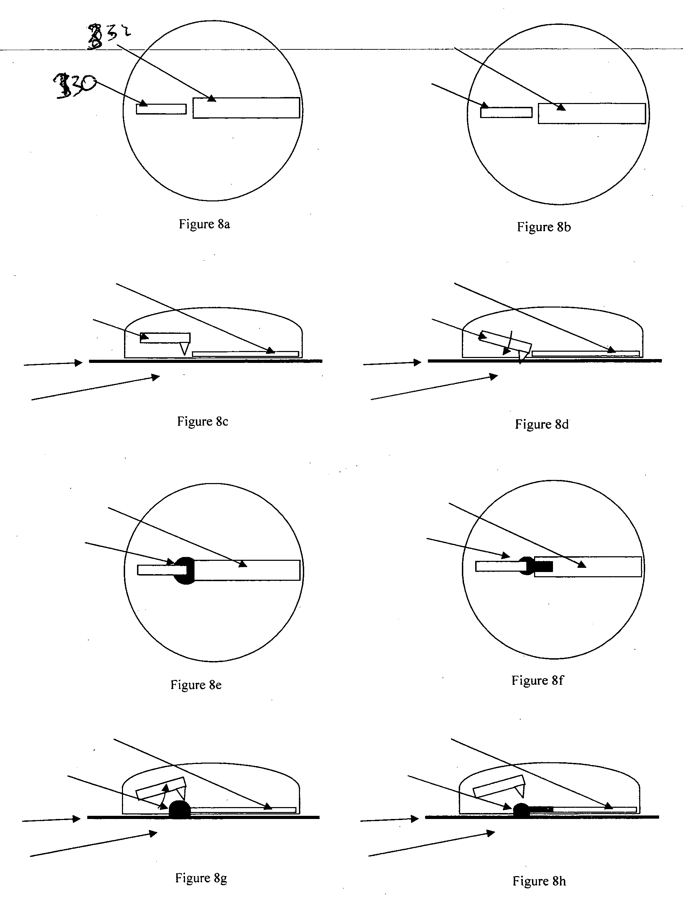

Figures 8A-H depict a method of sampling and measuring a bodily fluid with the analyte measuring device 300 of Figure 1A wherein the analyte measuring element 332 is stationary within housing 301. Figures 8a,8b,8e & 8f show top view of the device operation and Figures 8c,8d,8g & 8h show side view of the device operation. Figures 8a & 8c depicts piercing element 330 and analyte measurement element 332 prior piercing skin surface 306 through opening 310. Optionally and preferably, analyte measurement element 332 has a shape similar to a glucose test strip, as is well known in the art. Next, Figures 8b & 8d piercing element 330 is released preferably a spring mediated motion produces the downward movement of the piercing lancet (not shown) through opening 310 and pierces the skin and move up again. After the skin is pierced a fluid droplet enters into the base analyte monitoring unit and forms a droplet as shown at Figures 8e & 8g. Next, the fluid droplet accumulating through opening 310 comes into contact with analyte measurement element 332 and is taken into its measurement cavity via capillary action, allowing for a measurement to take place as is known and accepted in the art, as shown in Figures 8f & 8h.

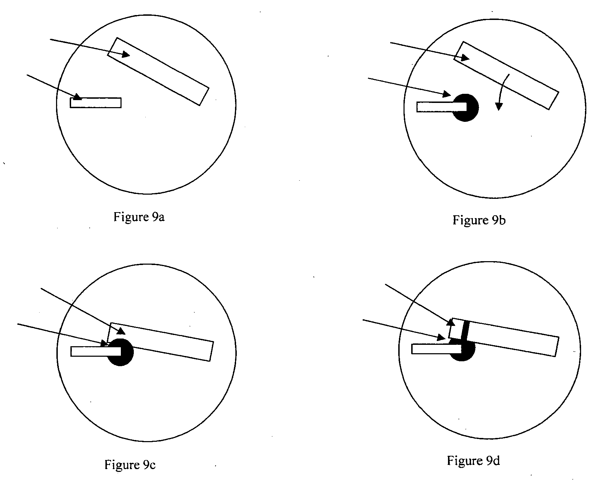

Figures 9A-D depict an optional method of sampling and measuring a bodily fluid with the analyte measuring device 300 of Figure 8D wherein at least one or more analytes measuring element 332 is coupled to movement module 350 utilizing rotation displacement. In Figure 9A piercing element 330 and at least one analyte measurement element 332 are shown prior to extracting a bodily fluid by piercing the skin with piercing element 330. Optionally and preferably, analyte measurement element 332 has a shape similar to a glucose test strip, as is well known in the art. Next in Figure 9B piercing element 330 is released preferably a spring mediated motion produces the downward movement of the piercing lancet (not shown) through opening 310 and pierces the skin and move up again. Most preferably this downward motion creates a single pierce in the skin surface 306. Next, following the skin piercing a bodily fluid droplet collects at the base of analyte monitoring device 300 through opening 310. During the piercing process analyte measurement element 322 is displaced optionally with movement module 350 from it is initial position within housing 301 toward the accumulated droplet of bodily fluid pooling at opening 310. Next, Figure 9c, analyte measurement element 322 arrives at opening 310 which is at the side of the strip, as the configuration of some of know in the art glucose test strips. and is in contact with bodily fluid. Next, Figure 9D bodily fluid droplet accumulates in opening 310 diffuses to analyte measurement element 332 preferably by capillary force allow device 300 to measure the relative levels of analyte within the bodily fluid for example including but not limited to blood and/or interstitial fluid. For example, analyte measuring element 332 may optionally be in the form of a glucose test strip measuring that blood glucose levels.

Optionally, the method described in figures 9A-D may be adapted to accommodate at least one or more measurement element 332. Optionally this may facilitated by timing the movement of a first measurement element 332 relative to a second measurement element, therein providing for sequential analyte measurement of a bodily fluid.

Optionally and preferably the method described in figures 9A-Dand the timing of movement element 350 may be controlled so as to optimize the likelihood of a good measurement optionally by allowing for a sufficient time lag between piercing and sampling preferably to allow sufficient bodily fluid build up, Figure 9B.

Optionally and preferably the method described in figures 9A-Dand the timing of movement element 350 may be controlled so as to optimize the likelihood of a good measurement optionally by correlating movement with at least one or more sensor comprised in analyte measuring device 300 of Figure 1. Most preferably, a blood pooling sensor provides additional control for triggering and coordinating the activity of movement element 350 based on the level of blood pooled at opening 310 of Figure 1. Optionally blood pooling sensor comprises an optical sensor utilizing the hemoglobin light absorption properties to determine if sufficient blood and/or bodily fluid is available for measuring.

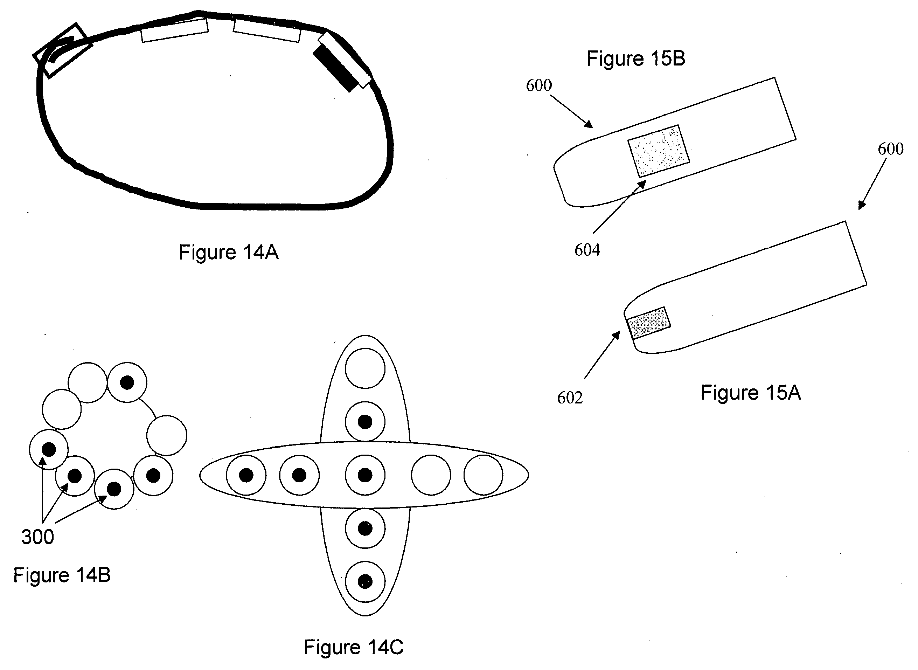

. Optionally and preferably the method described in figures 9A-Dand the timing of movement element 350 of Figure 1 may be adapted with respect to the analyte measuring element 332 utilized. For example a specialized analyte measurement element 600 according to an optional embodiment of the present invention as shown in Figure 15A, showing top view. Analyte measuring element 600 may be moved into a first position for uptake of the exposed bodily fluid using capillary action into measurement channel 602, as described and shown in Figure 9C. While an additional movement optionally provided by movement element 350 of Figure 1 may be utilized to advance measurement element 600, bottom view of Figure 15B, to a second position along its length comprising a blood coagulation element 604, as depicted in Figure 15B. Most preferably, blood coagulation element 604 prevents excess bleeding at the piercing site. Optionally blood coagulation element may take various forms for example including but not limited to a gauze pad, band aid, medicated pad with a coagulating agent, or the like elements for preventing excess blood loss and absorbing excess pooled blood.

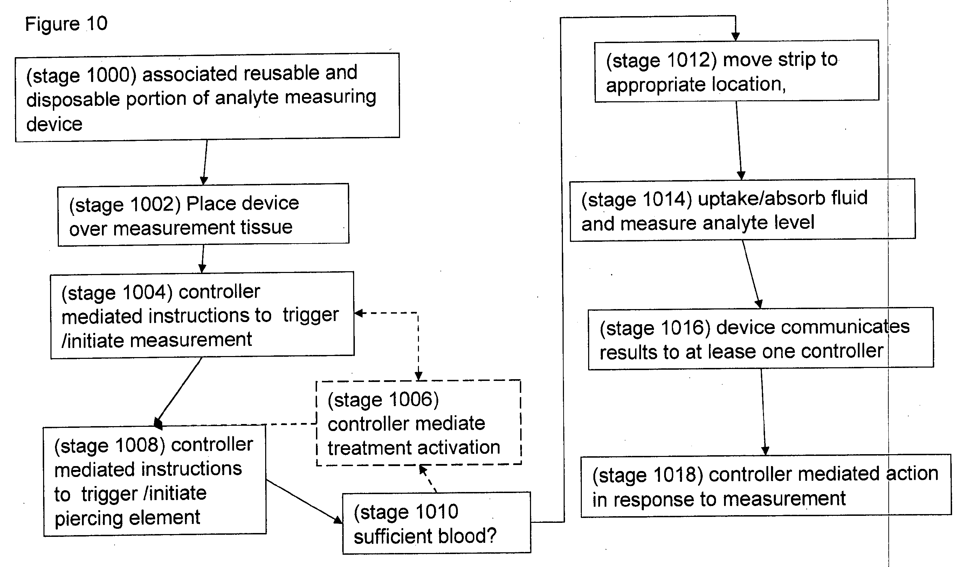

Figure 10 provides a flow chart depicting a preferred method for performing an analyte measurement with the optional embodiments for analyte measuring device as described in Figures 1-7 and providing a flow chart for the methods visually depicted in figure s 8 and 9.

In stage 1000 the disposable and reusable portions of the analyte measuring device are securely associated with one another to render the analyte measuring device functional and ready for use. Next, in stage 1002 the analyte measuring device is attached or otherwise coupled to a user of a measuring site to await a triggering signal preferably from a controller, optionally from at least one of the remote controller unit and/or a proximal controller unit. Optionally, the site and manner in which it is associate depends on the device utilized according to an optional embodiment.

Next in stage 1004 a triggering signal is received from a controller, optionally a controller may indicate to a user to manually trigger or initiate measurement. Next in stage 1006 optionally a treatment element is activated to increase blood perfusion at the targeted piercing site. Optionally, the treatment may include mechanical massage, heat, ultrasound, delivery of energy, delivery of electrical energy, drug delivery, or the like treatment to improve blood circulation in and around the targeted piercing site. Next in stage 1008, the piercing element is put into action to pierce the underlying tissue releasing a bodily fluid most preferably blood. Optionally and preferably the timing is controller mediated optionally by implementing a predetermined and controllable delay from the triggering signal of stage 1002 or time of the day or optionally with a second triggering signal for piercing activation communicated by at least one controller to the reusable portion of the analyte measuring device. Next in stage, 1010 a sensor preferably with the reusable portion and optionally in the disposable portion determines if sufficient blood volume has been released. Optionally and preferably blood volume determination is mediated with communication with the controller or optionally by local controls within the reusable portion of the measuring device.

Optionally if it is determined that there is an insufficient amount of blood volume for measurement to take place, optionally and preferably the controller initiates an appropriate treatment protocol to increase blood volume, as described in stage 1006. Optionally, instead of measuring the amount of extracted blood at this stage, a predetermined delay between piercing and measurement, for example 7 seconds is set in order to have high likelihood that enough blood for accurate measurement was pooled. Optionally if it is determined that there is sufficient blood volume to allow for a meaningful measurement and analysis the controller signals or triggers the activity of the movement module preferably as described in movement module 350 of Figure 1, to move the measuring element into place, in stage 1012. In stage 1014 the reusable portion is utilized to analyze and measure the analyte from the bodily fluid sample, most preferably blood, and once determined the information is communicated to at least one or more controllers for further analysis. Next in stage 1016 the controller analyses the measuring data and determines if any further action is necessary, undertaken in stage 1018, for example if blood glucose levels are sufficient or if not within acceptable range to sound an alarm, and/or undertake further actions necessary for the control and maintenance of appropriate analyte levels within the bodily fluid.

The analyte measuring devices described above in Figures 1-7 provide an analyte measuring device that optionally functional in an independent manner however most preferably and according to a preferred embodiment analyte measuring device described above in Figure 1-7 may function in an interactive and modular fashion able to interface with a plurality of similar analyte measuring devices, as well as auxiliary device to bring about closed loop control of analyte measurement and monitoring. For Example, a closed loop control as applied to diabetes comprises continuously measurement of glucose level, while responding to the measured level by administrating the prescribed amount of insulin and or additional treatment, for example with a pump. Such a closed loop control system is said to be an artificial pancreases.

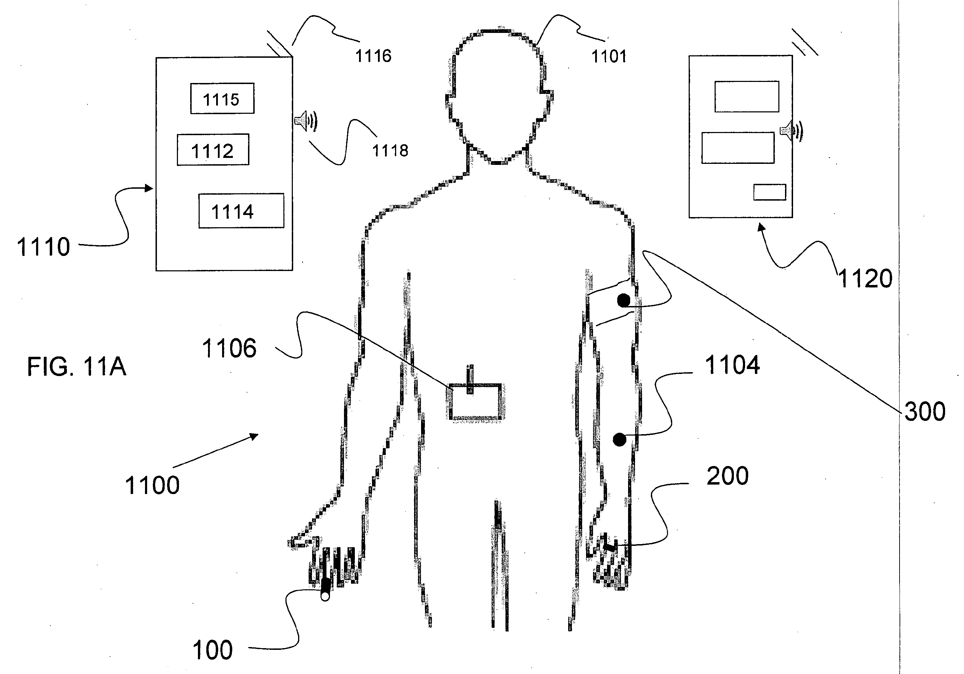

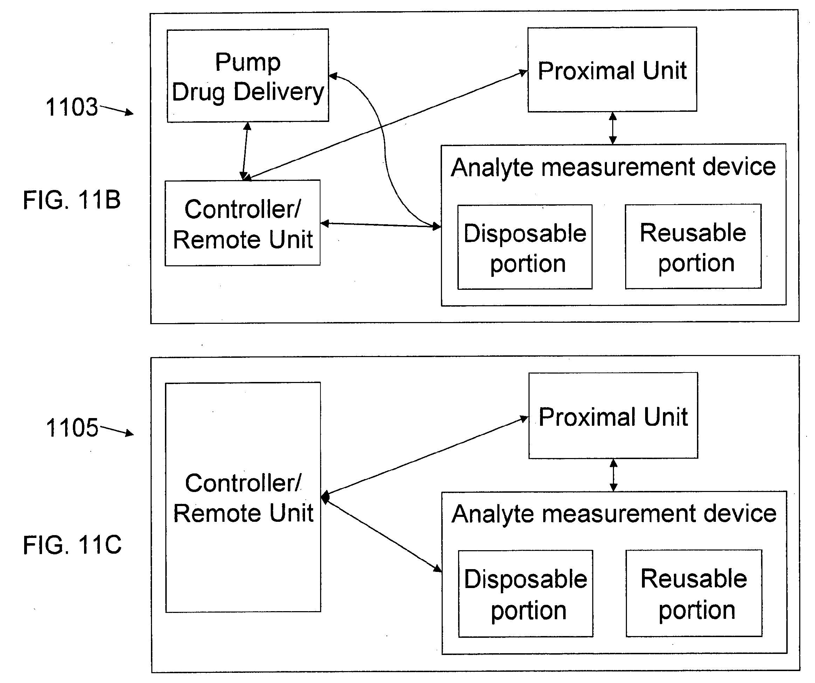

An optional embodiment comprises a modular system for providing closed loop control of an analyte monitoring system, for example including but not limited to glucose, cholesterol, triglycerides or the like analyte measurable from a bodily fluid most preferably blood and or interstitial fluid. Figure 11 provides a depiction

A preferable embodiment is the analyte monitoring system of the present invention that is specifically adapted for monitoring the analyte level, most preferably glucose, during sleep. More specifically as applied for the younger individuals where diabetes management is difficult particularly at night.

Figure 11 depicts system 1100 comprising a user 1101 is equipped with modular system 1101 comprising a plurality of modular analyte measuring devices for example a first device 100 described in Figure 5A-I that disposed over a finger; a second modular ring shaped analyte measuring device 200 as described in Figure 6A-C disposed over a finger; a third analyte measuring device 300 as described in Figure 1A-D; a drug delivery device and/or pump depicted as an insulin pump 1106; and a controllers 1110 and/or 1120. Most preferably, controller 1110 synchronizes the functioning of each of the components of the system. Preferably, controller 1110 coordinates to ensures that glucose sampling, measurements, in a timely and/or organized manner to maintain a stable blood sugar level over a period of time such as during the night sleep. Optionally the system includes also a subcutaneous continuous glucose sensor 1104 which can provide together with the ABGS system an accurate enough glucose monitor which can support closed loop control of the insulin delivery device and provide a stable blood sugar level over an extended continuous period of time preferably at least 8 hours continuously during night sleep, more preferably up to 16 hrs, and most preferably virtually continuously up to about 24 hours.

Most preferably each of the components of the closed loop system are in continuously communication using communication protocols as is known and accepted in the art.

Optionally and preferably, controller 1120 preferably comprises a display 11120, user interface 1114, speakers 1116, communication module1118, as well as at least one or more sync port modules 1115. Preferably, sync port module allows to physically interact and/or associate with other system devices for example including but not limited to optional analyte monitor devices as described in figures 1-7, for example reusable portion 820 depicted in Figure 3A-C. Optionally, communication module 1118 provides for communicating between the individual system component as well as external communication for example to a call center, hospital, health care provider or the like. Preferably, speaker 1116 provides for audible cues to be communicated for example sounding an alarm when analyte levels drop below a particular threshold.
Optionally and preferably, user interface 1114 optionally in the form of a keyboard, keypad, and/or touch screen allows a user to interface directly with the device and the various components, for example, setting timing of drug delivery or of measurements, schedules or the like.

Optionally system 1110 may be realized with one or more controllers. Optionally, a first controller may be configured to be a primary controller 1110 also referred to as a remote controller located at a distance from user 1101. Optionally and preferably as second controller unit 1120 and proximally located near user 1101, also referred to as proximal controller 1120 is similarly used to communicate with and coordinate between the plurality of measuring devices 100, 300, 1104 and 200 defined and used within the system. Most preferably proximal controller 1120 is close to user 1101 and is in communication with using near range communication protocols for example including but not limited to wireless, Bluetooth, contactless RFID or the like. Optionally and preferably proximal controller 1120 comprises long range communication ability for example including but not limited to cellular, wireless, optical or the like long range communication protocols as is known and accepted in the art. Optionally and preferably, proximal controller 1120 may comprise the same components and interface as remote controller 1110. Optionally, proximal controller 1120 and remote controller 1110 may be associated with one another. Optionally, proximal controller 1120 and remote controller 1110 may be interchangeable with one another.

Figure 11B provides a schematic block diagram of system 1101.

Figure 11C provides a schematic block diagram of an optional system 1105 according to the present invention wherein the system does not comprise a drug delivery device.

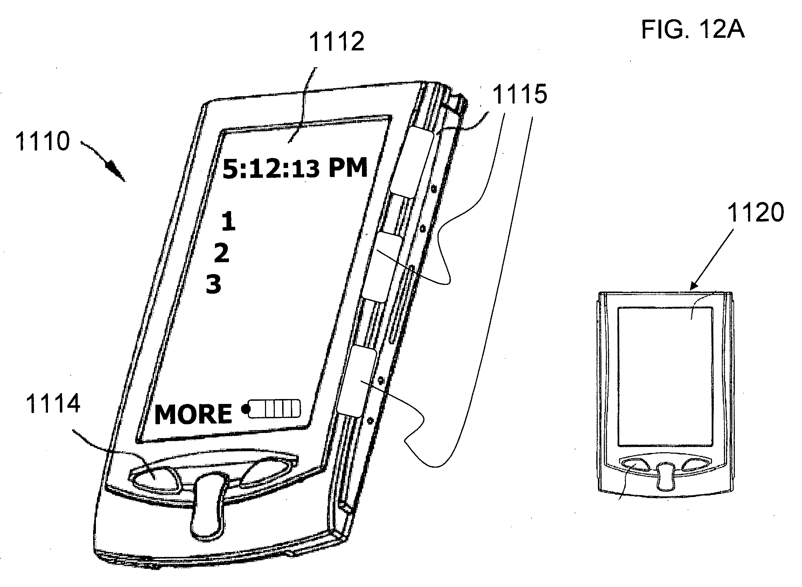

Figures 12A-D provide a schematic depiction of optional the modular devices that may be used interchangeably with the analyte measuring device. Figure 12A provides a schematic diagram of the a modular centralized controllers 1110, and 1120 as described in Figure 11A in the from of a PDA and/or mobile telephone. Controllers 1120 and 1110 preferably comprise a display 1112, user interface 1114, a plurality of sync ports 1115 wherein the reusable portion of the analyte measuring device may be placed for a plurality of functions for example including but not limited to recharging the reusable part, data exchange, synchronization, storage or the like. Preferably, user interface 1114 may be utilized to program controller 1110 to schedule of tests of several modular analyte measuring devices as depicted in Figures 1-7 according to input information, preset schedule or other inputs. Optionally, controller 1110 and/or 1120 may further comprise ports and or interface for example including but not limited to USB to provide for linking and exchanging information with a plurality of devices not shown. Optionally and preferably display 1112 depicts the status of each of the analyte measuring devices associated with the device. For example, display 1112 may depict when a measurement is scheduled to take place, active status, last reading, alarm, graphical data relating to the user or the like information.

Optionally, controller 1120 may be realized as an identical unit to controller 1110 however comprising a master/slave controller hierarchy. Optionally, controller 1120 may be realized as a small unit comprising only essential components for example including but not limited to communication module (not shown) and display.

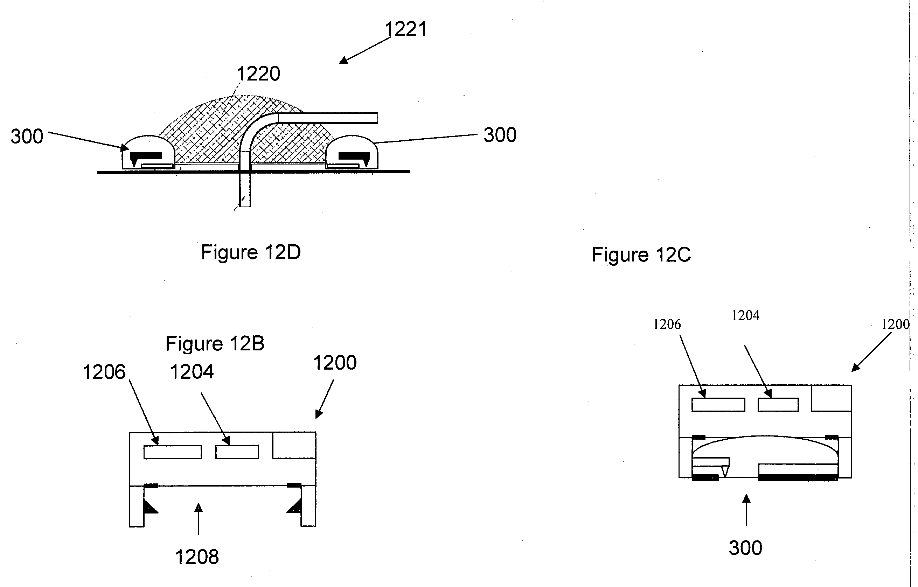

Figure 12B provides a schematic depiction of a controller 1200 that forms part of the reusable portion of the modular analyte measuring devices (not shown) as depicted in Figures 1-7. Controller 1200 comprising power source 1202 , transponder 1204 for contactless communication, controller 1206 and further comprising a recess 1208 to accept disposable portion of modular analyte measuring devices (not shown) as depicted in Figures 1-7.

Figure 12 C shows a schematic optional configuration of the disposable and reusable portions of a modular analyte measuring device 300 as described in Figure 1 that is physically synchronizing with controller 1200. Optionally during synchronization, data may be exchanged, sync may serve to replenish battery or other power resources.

Figure 12D shows an example of the modular nature of analyte measuring device. In this example two modular analyte measuring devices 300 as described in Figure 1 are attached to the securing element of an insulin infusion set 1220. Optionally, the modular analyte measuring device 300 can also communicate with the insulin pump, such as the insulin pump can act as the previously described controller. In this case for instance the insulin pump can activate ABGS measurement during the night and in case of risk for hypoglycemia suspend the Basel insulin delivery. In case it is not enough the insuln pump can alert also the user or other users. In another option a continuous subcutaneous glucose monitor 1104, as shown in Figure 11A, optionally with an insulin pump(not shown) that is coupled to two modular analyte measuring devices 300 as described in Figure 1. Most preferably, the modular nature of device 300 allows it to be in continuous communication with a plurality of devices including a second analyte measuring device 300 or a subcutaneous glucose monitor 1220 to create an alternative closed loop system without the need for a centralized controller, as depicted in Figure 11. Optionally and preferably system 1221 may serve as a sensor calibration tool adept for calibrating interstitial tissue readings.

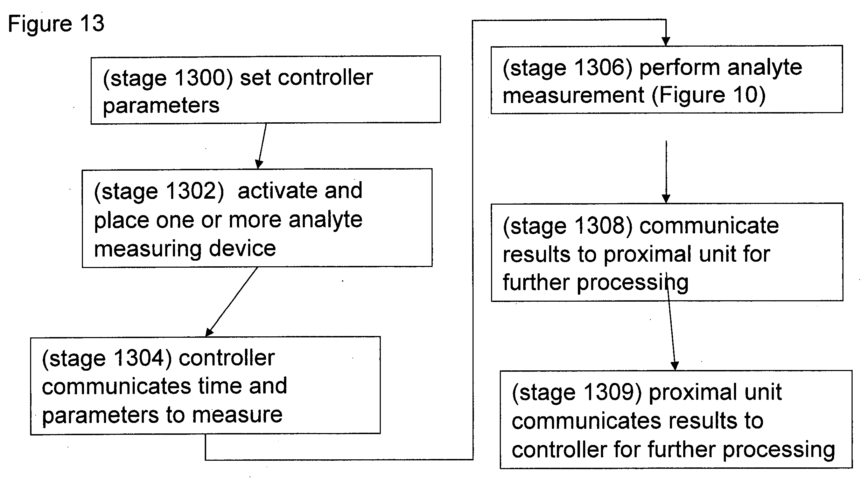

Figure 13 provides a flow chart of a method for monitoring an analyte with the modular analyte measurement device, as described above and depicted in Figures 1-7 above comprising a remote controller and a proximal controller. The method of figure 13 is particularly adept for monitoring glucose at night and more specifically for kids as it provides with remote control of the measuring and monitoring processes. A preferable system and method provided for both remote and local control that is adapted for adolescents, older children, mildly handicapped, elderly to have some control over the measuring and monitoring process they depend upon. Optionally a system and method comprising only a single remote controller may be best suited for infants, small children, severely handicapped or injured that require external monitoring. Optionally the method of depicted in Figure 13 may be adapted for monitoring and measuring an analyte at night or alternatively during the day.

In stage 1300 the controllers are set and loaded with the appropriate data for example including the number of analyte measuring devices used, most preferably one per each measurement required in a given time period. For example during nigh sleep if three measurements are required then three analyte measuring device will be utilized, where in stage 1300 the timing of each will be set. Next in stage 1302 the analyte measuring device of choice is placed in the measuring area, most preferably extremities such as the arms, legs, fingers and or toes. The device configuration used depends on the locations. Next the controller and the measuring devices communicate and begin to countdown until the time for measurement occurs. Optionally, the trigger for measurement may be an auxiliary unit, for example a continuous glucose monitoring device, such as 1104 shown in Figure 11A. Next in stage 1304 the controller communicates to the reusable portion of the analyte measuring device to undertake a measurement. Most preferably, initiating the method described in Figure 10. Next in stage 1306 measurement is undertaken and optionally and preferably communicated to the proximal controller for further processing in stage 1308. Next in stage 1310 the proximal controlling unit communicate to the remote controlling unit the results and optionally any further action for example including alarm, activating a treatment element, activating a drug delivery device or the like. Most preferably once a first test is accomplished, the system return to stage 1302 awaiting the next scheduled measurement time.

Figures 14 A-C depict optional accessories that may be utilized with the analyte measuring device, for example 300, as described above and in particular in Figure 1 -7. Figure 14A provides a depiction of a quick release flexible strap that may be fit with a plurality of analyte measuring within predefined location along the strap. Figure 14B provides a depiction of a round flexible base wherein a plurality of analyte measuring devices, for example 300 of Figure 1, are disposed along its circumference. Optionally and preferably, this configuration may be combined with the insulin infusion set securing element as described in Fig. 12D. Figure 14C provides an additional depiction of flexible base for holding a plurality of analyte measuring devices.

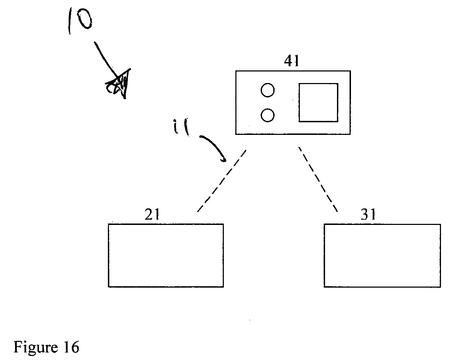

Figure 16 provides an additional optional embodiment of an optional system 10 for improved calibration of IGS sensors, as previously described in detail. System 10 comprising an IGS sensor 31, as known in the art, an ABGS 21, described in Figures 1-7, that are both wirelessly coupled with a controller 41, according to an optional embodiment as previously described in figures 11 and 12. Preferably controller 41 comprises a processor, user interface and display in a single unit which can be used to process data and initiate activation of at least one or both of ABGS 21 and IGS 31, while optionally coordinating the operational activity of both ABGS 21 and IGS sensor 31 preferably through wireless coupling shown in dashed communication lines 11, preferably comprising wireless communication channels. Optionally, wireless communication channel 11 may be realized according to at least one or more optional communication protocols as is known and accepted in the art for example including but not limited to, IR, optical, wireless, cellular, RF or the like communication protocols.. Most preferably, the centralized control and coordination by controller 41 of the present invention provides for an optional system and method for calibrating an IGS sensors as is known in the art with the additional blood glucose measuring device 31 provided by a preferred embodiment.

Figure 16 provides an example of a non limiting example for an optional embodiment .

While the invention has been described with respect to a limited number of embodiments, it will be appreciated that many variations, modifications and other applications may be made.

## Claims

1. A system for measuring an analyte from blood over a measuring area comprising:
a. a disposable housing (810, 801) having an opening for receiving the blood for measuring the analyte and at least two disposable electrical contacts (816, 818) for performing the measurement; wherein the disposable housing (801) comprises a single skin piercing element (830), a single measuring element (832) sensitive to the analyte from the blood and a coupling mechanism (816) for attaching the housing over the measuring area;
b. a reusable housing (820, 801) having at least two reusable electrical contacts (826, 828) corresponding to the at least two disposable electrical contacts (816, 818); and comprising a power source, a controller (840), a transponder and a mechanical actuator for performing and communicating the measurement;
c. wherein the reusable housing is associated with the disposable housing over the at least two corresponding reusable electrical contacts (826, 828) and the at least two disposable electrical contacts (816, 818); and wherein the mechanical actuator is coupled to and manipulates the single skin piercing element (830) and the single measuring element (832);
d. a remote controller adapted for wireless communication with the transponder for communicating a triggering signal;
e. wherein the system is adapted such that the triggering signal triggers the mechanical actuator to manipulate the movement of the single skin piercing element (830) and the single measuring element (832) therein triggering the measurement of the analyte within the reusable housing (820) and communicating the measurement to the remote controller; and
f. wherein the remote controller is further adapted to receive the analyte measurement and to display it in the form of a concentration of the analyte in the blood.

2. The system according to claim 1, wherein the remote controller is adapted to control more than a single combination of the first (810) and second (820) housing (801).

3. The system according to claims 1 or 2, where the remote controller can communicate the measurement to a second remote controller.

4. The system according to any one of claims 1-3, that is further adapted to provide alarm if the measurement concentration is out of a certain range.

5. The system according to any preceding claims, where the remote controller is a cellular phone.

6. The system according to claim 5, wherein the cellular phone (Slave) can be activated by a remote message (SMS) from a remote cellular phone (Master) sending the message to the number associated with the first cellular phone (Slave) and causing it to trigger the measurement.

7. The system according to any preceding claim, further comprising an adhesive layer (816) for coupling the housing (810, 801) to skin surface defining the measuring area chosen from the group consisting of arms, legs, shoulder, hip, back and abdomen.

8. The system according to any preceding claim, further comprising a communication module for receiving remote activation instructions and sending data related to the measurement.

9. The system according to claim 8, wherein the communication is achieved using communication protocols chosen from the group consisting of contact- less RFID, RF, wireless, cellular, IR.

10. The system according to any preceding claim, wherein the housing (801) further comprises at least one tissue treatment element (834) for improving analyte measurement and/or analysis.

11. The system according to claim 10, wherein the treatment element (834) is chosen from the group consisting of electrical energy, ultrasound energy, optical energy, acoustic energy, vasodilatation drugs, applied heating, massage, and mechanical energy.

12. The system according to any preceding claim, further comprising a movement module associated with the at least one or more analyte measuring element.

13. The system according to claim 12, wherein the movement module is adapted to displace the analyte measuring element from an initial position within the housing to a position that is in contact with the blood accumulating over the opening and further to a final position.

14. The system according to any preceding claim, wherein the reusable portion (820) comprises at least one or more analyte measuring sensors chosen from the group consisting of amperemetric, acoustic, ultrasonic, optical, electromagnetic, infrared.

## Patentansprüche

1. System zum Messen eines Analyten aus Blut über einem Messbereich, welches umfasst:
a. ein Einweggehäuse (810, 801), das eine Öffnung zum Aufnehmen des Blutes zum Messen des Analyten und wenigstens zwei wegwerfbare elektrische Kontakte (816, 818) zum Durchführen der Messung aufweist; wobei das Einweggehäuse (801) ein einziges Hautdurchbohrungselement (830), ein einziges Messelement (832), das gegenüber dem Analyten aus dem Blut empfindlich ist, und einen Kopplungsmechanismus (816) zum Befestigen des Gehäuses über dem Messbereich umfasst;
b. ein Mehrweggehäuse (820, 801), das wenigstens zwei wiederverwendbare elektrische Kontakte (826, 828) aufweist, die den wenigstens zwei wegwerfbaren elektrischen Kontakten (816, 818) entsprechen; und das eine Energiequelle, eine Steuereinrichtung (840), einen Transponder und einen mechanischen Aktuator zum Durchführen der Messung und Übermitteln des Messwertes umfasst;
c. wobei das Mehrweggehäuse mit dem Einweggehäuse über die wenigstens zwei entsprechenden wiederverwendbaren elektrischen Kontakte (826, 828) und die wenigstens zwei wegwerfbaren elektrischen Kontakte (816, 818) verbunden ist; und wobei der mechanische Aktuator mit dem einzigen Hautdurchbohrungselement (830) und dem einzigen Messelement (832) gekoppelt ist und diese betätigt;
d. eine Fernsteuereinrichtung, die für eine drahtlose Kommunikation mit dem Transponder ausgelegt ist, um ein Auslösesignal zu übermitteln;
e. wobei das System derart ausgelegt ist, dass das Auslösesignal den mechanischen Aktuator auslöst, um die Bewegung des Hautdurchbohrungselements (830) zu betätigen, und das einzige Messelement (832) darin die Messung des Analyten innerhalb des Mehrweggehäuses (820) auslöst und den Messwert zu der Fernsteuereinrichtung übermittelt; und
f. wobei die Fernsteuereinrichtung ferner dafür ausgelegt ist, den Analytenmesswert zu empfangen und ihn in der Form einer Konzentration des Analyten im Blut anzuzeigen.

2. System nach Anspruch 1, wobei die Fernsteuereinrichtung dafür ausgelegt ist, mehr als eine einzige Kombination des ersten (810) und zweiten (820) Gehäuses (801) zu steuern.

3. System nach Anspruch 1 oder 2, wobei die Fernsteuereinrichtung den Messwert zu einer zweiten Fernsteuereinrichtung übermitteln kann.

4. System nach einem der Ansprüche 1-3, welches ferner dafür ausgelegt ist, einen Alarm bereitzustellen, falls die gemessene Konzentration außerhalb eines gewissen Bereichs liegt.

5. System nach einem der vorhergehenden Ansprüche, wobei die Fernsteuereinrichtung ein Mobiltelefon ist.

6. System nach Anspruch 5, wobei das Mobiltelefon (Slave) durch eine Fernnachricht (SMS) von einem entfernten Mobiltelefon (Master) aktiviert werden kann, das die Nachricht an die Nummer sendet, die dem ersten Mobiltelefon (Slave) zugeordnet ist, und bewirkt, dass dieses die Messung auslöst.

7. System nach einem der vorhergehenden Ansprüche, welches ferner eine Klebeschicht (816) zum Koppeln des Gehäuses (810, 801) mit einer den Messbereich definierenden Hausfläche umfasst, die aus der Gruppe ausgewählt ist, welche aus Armen, Beinen, Schulter, Hüfte, Rücken und Unterleib besteht.

8. System nach einem der vorhergehenden Ansprüche, welches ferner ein Kommunikationsmodul zum Empfangen von Fernaktivierungs-Anweisungen und Senden von Daten, welche die Messung betreffen, umfasst.

9. System nach Anspruch 8, wobei die Kommunikation unter Verwendung von Kommunikationsprotokollen erzielt wird, die aus der Gruppe ausgewählt sind, welche aus kontaktlosen RFID-, RF-, Wireless-, Mobilfunk-, IR-Protokollen ausgewählt sind.

10. System nach einem der vorhergehenden Ansprüche, wobei das Gehäuse (801) ferner wenigstens ein Gewebebehandlungselement (834) zum Verbessern der Messung und/oder Analyse des Analyten umfasst.

11. System nach Anspruch 10, wobei das Behandlungselement (834) aus der Gruppe ausgewählt ist, welche aus elektrischer Energie, Ultraschallenergie, optischer Energie, akustischer Energie, Medikamenten zur Vasodilatation, Zuführen von Wärme, Massage und mechanischer Energie besteht.

12. System nach einem der vorhergehenden Ansprüche, welches ferner ein Bewegungsmodul umfasst, das dem wenigstens einen oder mehreren Analyten-Messelementen zugeordnet ist.

13. System nach Anspruch 12, wobei das Bewegungsmodul dafür ausgelegt ist, das Analyten-Messelement von einer Anfangsposition innerhalb des Gehäuses zu einer Position, in der es sich in Kontakt mit dem sich über der Öffnung ansammelnden Blut befindet, und weiter zu einer Endposition zu verlagern.

14. System nach einem der vorhergehenden Ansprüche, wobei der wiederverwendbare Abschnitt (820) wenigstens einen oder mehrere Analyten-Messsensoren umfasst, die aus der Gruppe ausgewählt sind, welche aus Strommess-, akustischen, Ultraschall-, optischen, elektromagnetischen und Infrarotsensoren besteht.

## Revendications

1. Système destiné à mesurer un analyte sanguin sur une zone de mesure, comprenant :
a. un boîtier à usage unique (810, 801) présentant une ouverture destinée à recevoir le sang pour mesurer l'analyte, et au moins deux contacts électriques à usage unique (816, 818) pour effectuer la mesure ; dans lequel le boîtier à usage unique (801) comprend un unique élément de perforation de la peau (830), un unique élément de mesure (832) sensible à l'analyte sanguin, et un mécanisme de couplage (816) destiné à fixer le boîtier sur la zone de mesure ;
b. un boîtier réutilisable (820, 801) présentant au moins deux contacts électriques réutilisables (826, 828) correspondant auxdits au moins deux contacts électriques à usage unique (816, 818) ; et comprenant une source d'alimentation, un contrôleur (840), un transpondeur et un actionneur mécanique pour effectuer et communiquer la mesure ;
c. dans lequel le boîtier réutilisable est associé au boîtier à usage unique sur lesdits au moins deux contacts électriques réutilisables correspondants (826, 828) et lesdits au moins deux contacts électriques à usage unique (816, 818) ; et dans lequel l'actionneur mécanique est couplé à, et manipule, l'unique élément de perforation de la peau (830) et l'unique élément de mesure (832) ;
d. un contrôleur distant apte à mettre en oeuvre une communication sans fil avec le transpondeur en vue de communiquer un signal de déclenchement ;
e. dans lequel le système est adapté de sorte que le signal de déclenchement déclenche l'actionneur mécanique afin de manipuler le mouvement de l'unique élément de perforation de la peau (830) et l'unique élément de mesure (832) dans celui-ci, déclenchant ainsi la mesure de l'analyte au sein du boîtier réutilisable (820) et communiquant la mesure au contrôleur distant ; et
f. dans lequel le contrôleur distant est en outre apte à recevoir la mesure d'analyte et à l'afficher sous la forme d'une concentration de l'analyte dans le sang.

2. Système selon la revendication 1, dans lequel le contrôleur distant est apte à commander plus d'une combinaison unique du premier (810) et du second (820) boîtier (801).

3. Système selon la revendication 1 ou 2, dans lequel le contrôleur distant peut communiquer la mesure à un second contrôleur distant.

4. Système selon l'une quelconque des revendications 1 à 3, lequel est en outre apte à fournir une alarme si la concentration de mesure est en dehors d'une certaine plage.

5. Système selon l'une quelconque des revendications précédentes, dans lequel le contrôleur distant est un téléphone cellulaire.

6. Système selon la revendication 5, dans lequel le téléphone cellulaire (esclave) peut être activé par un message à distance (SMS) en provenance d'un téléphone cellulaire distant (maître) qui envoie le message au numéro associé au premier téléphone cellulaire (esclave) et l'amène à déclencher la mesure.

7. Système selon l'une quelconque des revendications précédentes, comprenant en outre une couche d'adhésif (816) pour coupler le boîtier (810, 801) à la surface de la peau définissant la zone de mesure choisie à partir du groupe comportant les bras, les jambes, l'épaule, la hanche, le dos et l'abdomen.

8. Système selon l'une quelconque des revendications précédentes, comprenant en outre un module de communication destiné à recevoir des instructions d'activation à distance et à envoyer des données connexes à la mesure.

9. Système selon la revendication 8, dans lequel la communication est réalisée en utilisant des protocoles de communication choisis à partir du groupe comportant les protocoles RFID sans contact, RF, sans fil, cellulaire, IR.

10. Système selon l'une quelconque des revendications précédentes, dans lequel le boîtier (801) comprend en outre au moins un élément de traitement de tissus (834) pour améliorer une analyse et/ou une mesure d'analyte.

11. Système selon la revendication 10, dans lequel l'élément de traitement (834) est choisi à partir du groupe comportant de l'énergie électrique, de l'énergie ultrasonore, de l'énergie optique, de l'énergie acoustique, des vasodilatateurs, un chauffage appliqué, un massage et de l'énergie mécanique.

12. Système selon l'une quelconque des revendications précédentes, comprenant en outre un module de mouvement associé à au moins un ou plusieurs éléments de mesure d'analyte.

13. Système selon la revendication 12, dans lequel le module de mouvement est apte à déplacer l'élément de mesure d'analyte, depuis une position initiale au sein du boîtier jusqu'à une position qui est en contact avec le sang s'accumulant sur l'ouverture, et en outre jusqu'à une position finale.

14. Système selon l'une quelconque des revendications précédentes, dans lequel la partie réutilisable (820) comprend au moins un ou plusieurs capteurs de mesure d'analyte choisis à partir du groupe comportant des capteurs ampérométriques, acoustiques, ultrasonores, optiques, électromagnétiques, infrarouges.
